(19) **Europäisches Patentamt**
**European Patent Office**
**Office européen des brevets**

(11) **EP 2 393 423 B1**

(12) **EUROPEAN PATENT SPECIFICATION**

(45) Date of publication and mention
of the grant of the patent:
**31.07.2019 Bulletin 2019/31**

(51) Int Cl.:
**A61B 5/00** $^{(2006.01)}$

(21) Application number: **10739047.8**

(22) Date of filing: **03.02.2010**

(86) International application number:
**PCT/US2010/023001**

(87) International publication number:
**WO 2010/091055 (12.08.2010 Gazette 2010/32)**

(54) **CALCULATING CARDIOVASCULAR PARAMETERS**

BERECHNUNG VON KARDIOVASKULÄREN PARAMETERN

CALCUL DE PARAMÈTRES CARDIOVASCULAIRES

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **09.02.2009 US 150991 P**
**27.01.2010 US 695030**

(43) Date of publication of application:
**14.12.2011 Bulletin 2011/50**

(73) Proprietor: **Edwards Lifesciences Corporation
Irvine, CA 92614 (US)**

(72) Inventors:
• **HATIB, Feras
Irvine, CA 92614 (US)**
• **ROTELIUK, Luchy, D.
Irvine, CA 92614 (US)**

(74) Representative: **Eisenführ Speiser
Patentanwälte Rechtsanwälte PartGmbB
Postfach 31 02 60
80102 München (DE)**

(56) References cited:
WO-A2-2007/066343 WO-A2-2009/099833
US-A1- 2001 016 690 US-A1- 2005 124 904
US-A1- 2007 276 275 US-A1- 2008 015 451

• Benjamin Pratt ET AL: "Calculating Arterial
Pressure-Based Cardiac Output Using a Novel
Measurement and Analysis Method", , 1 January
2007 (2007-01-01), pages 403-411, XP055086642,
online Retrieved from the Internet:
URL:http://www.researchgate.net/publicatio
n/5853603_Calculating_arterial_pressure-ba
sed_cardiac_output_using_a_novel_measureme
nt_and_analysis_method/file/79e415097ff64b
8867.pdf [retrieved on 2013-11-04]
• MARIA GABRIELLA COSTA ET AL: "Continuous
and intermittent cardiac output measurement in
hyperdynamic conditions: pulmonary artery
catheter vs. lithium dilution technique",
INTENSIVE CARE MEDICINE, SPRINGER,
BERLIN, DE, vol. 34, no. 2, 6 October 2007
(2007-10-06), pages 257-263, XP019583285, ISSN:
1432-1238

EP 2 393 423 B1

Description

BACKGROUND

Indicators such as stroke volume (SV), cardiac output (CO), end-diastolic volume, ejection fraction (EF), stroke volume variation (SVV), pulse pressure variation (PPV), and systolic pressure variations (SPV), among others, are important not only for diagnosis of disease, but also for "real-time," i.e., continual, monitoring of clinically significant changes in a subject. For example, health care providers are interested in changes in preload dependence, fluid responsiveness, or volume responsiveness as well as cardiac output in both human and animal subjects. Few hospitals are therefore without some form of equipment to monitor one or more cardiac indicators in an effort to provide a warning that one or more of the indicated changes are occurring in a subject. Many techniques, including invasive techniques, non-invasive techniques, and combinations thereof, are in use and even more have been proposed in the literature.

Documents US 2005/124904 A1, US 2008/015451 A1 and the article "Calculating Arterial Pressure-Based Cardiac Output Using a Novel Measurement and Analysis Method" by Benjamin Pratt et al, Biomedical Instrumentation & Technology: September 2007, Vol. 41, No. 5, pp. 403-411, disclose multivariate statistical models to determine a cardiovascular parameter, but do not distinguish between normal and hyperdynamic patients.

The article "Continuous and intermittent cardiac output measurement in hyperdynamic conditions: pulmonary artery catheter vs. lithium dilution technique" by Maria Gabriella Costa et al, Intensive Care Medicine, vol. 34, no. 2, pages 257-263, Springer, Berlin, discloses cardiac output determination in hyperdynamic patients, but does not disclose how to distinguish between normal and hyperdynamic waveforms and to automatically apply different models.

SUMMARY

The invention is defined in the appended claims.

Methods for calculating cardiovascular parameters in a subject are described. These methods involve providing a subject's arterial pressure waveform data and analyzing the arterial pressure waveform data to determine if the subject is experiencing an abnormal condition. If the subject is determined to be experiencing the abnormal condition, then a multivariate statistical model is applied to the arterial pressure waveform data to determine the subject's cardiovascular parameter. This multivariate statistical model, for example, can be developed using data from subjects that were experiencing the abnormal condition. If the subject is not determined to be abnormal (i.e., the subject is experiencing the normal counterpart to the abnormal condition), then a multivariate statistical model is applied to the arterial pressure waveform data to determine the subject's cardiovascular parameter. This multivariate statistical model, for example, can be developed using data from subjects that were not experiencing the abnormal condition (i.e., subjects experiencing the normal counterpart to the abnormal condition).

Additionally, methods for measuring a cardiovascular parameter in hyperdynamic and non-hyperdynamic subjects are described. These methods involve providing a subject's arterial pressure waveform data and analyzing the arterial pressure waveform data to determine if the subject's peripheral arterial pressure is decoupled from the subject's central aortic pressure. Analyzing the arterial pressure waveform data to determine if the subject's peripheral arterial pressure is decoupled from the subject's central aortic pressure can be accomplished, for example, by using a multivariate Boolean model created based on data from both subject's experiencing decoupling and subjects experiencing normal hemodynamic conditions. If the subject's peripheral arterial pressure is determined to be decoupled from the subject's central aortic pressure, then the arterial pressure waveform data is analyzed to determine if the subject is hyperdynamic. Analyzing the arterial pressure waveform data to determine if the subject is hyperdynamic can be accomplished, for example, by using a multivariate Boolean model created based on data from both hyperdynamic subjects and subjects experiencing normal hemodynamic conditions. If the subject is determined to be hyperdynamic, then a multivariate statistical model is applied to the arterial pressure waveform data to determine the subject's hyperdynamic and decoupled cardiovascular parameter. This multivariate statistical model, for example, can be developed using data from subjects that were experiencing hyperdynamic conditions and decoupling. However, if the subject's peripheral arterial pressure is not determined to be decoupled from the subject's central aortic pressure or the subject is not determined to be hyperdynamic, then a multivariate statistical model is applied to the arterial pressure waveform data to determine the subject's normal cardiovascular parameter. This multivariate statistical model, for example, can be developed using data from subjects that were not experiencing decoupling or hyperdynamic conditions (i.e., subjects experiencing the normal hemodynamic condition).

DESCRIPTION OF DRAWINGS

Fig. 1 shows simultaneously recorded pressure waveforms in the ascending aorta (Aortic), femoral artery (Femoral), and radial artery (Radial) in a porcine animal model during normal hemodynamic conditions.

Fig. 2 shows simultaneously recorded pressure waveforms in the ascending aorta (Aortic), femoral artery (Femoral), and radial artery (Radial) in a porcine animal model during Endotoxin shock (septic shock) resuscitated with large amounts of fluids and vasopressors.

Fig. 3 shows a block diagram illustrating an example of logic for a method as described herein for determining whether to apply a normal multivariate statistical model to determine a cardiovascular or whether a multivariate statistical model based on abnormal conditions should be used.

Fig. 4 shows a block diagram illustrating an example of logic for a method as described herein for determining whether to apply a normal multivariate statistical model to determine arterial tone or whether a hyper dynamic multivariate statistical model should be used.

Fig. 5 shows an example of a complex blood pressure curve over one beat-to-beat heart cycle.

Fig. 6 shows a discrete-time representation of the pressure waveform of Fig. 5.

Fig. 7 shows the area under the systolic portion of the arterial pressure waveform.

Fig. 8 shows the statistical distributions of the area under the systolic phase of the arterial pressure waveform for normal subjects and hyperdynamic subjects.

Fig. 9 shows the duration of the systole for an arterial pressure waveform.

Fig. 10 shows the statistical distribution of the duration of the systole of the arterial pressure waveform for normal subjects and hyperdynamic subjects.

Fig. 11 shows the duration of the systole and the duration of the diastole for an arterial pressure waveform.

Fig. 12 is the statistical distribution of the duration of the diastolic phase for high heart rate subjects in normal hemodynamic conditions (dashed line) and hyperdynamic conditions (thick line)-the distribution for all the patients combined is also shown (thin line).

Fig. 13 is the statistical distribution of the duration of the systolic phase for high heart rate subjects in normal hemodynamic conditions (dashed line) and hyperdynamic conditions (thick line)-the distribution for all the patients combined is also shown (thin line).

Fig. 14 is a block diagram illustrating one example of a system for implementing the methods described herein.

## DETAILED DESCRIPTION

Methods for measuring a cardiovascular parameter, e.g., arterial tone, in a subject regardless of whether the subject is experiencing normal hemodynamic or abnormal hemodynamic conditions are described. These methods involve the determination of whether a subject is experiencing normal hemodynamic conditions or abnormal hemodynamic conditions, then applying an appropriate model to subject data to determine a cardiovascular parameter for the subject. In the methods described herein, separate multivariate statistical models are created for subjects experiencing abnormal hemodynamic conditions versus subjects experiencing normal hemodynamic conditions because, once a subject is experiencing the abnormal hemodynamic conditions, models based on subjects experiencing the normal hemodynamic conditions are not accurate. Having correct cardiovascular parameter values for a subject experiencing abnormal hemodynamic conditions, for example, enables the calculation of accurate values for parameters based upon the cardiovascular parameters in question, which in turn enable a clinician to appropriately provide treatment to the subject.

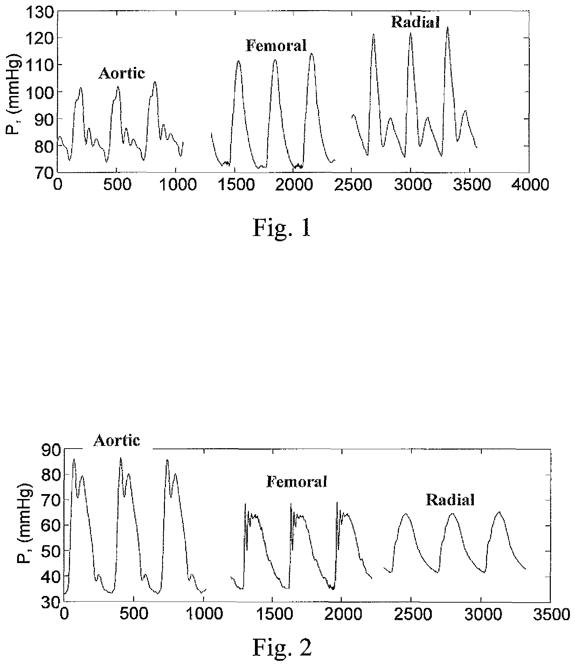

As a specific example, the methods described herein can be used to determine arterial tone in a subject regardless of whether the subject is experiencing normal hemodynamic conditions or hyperdynamic hemodynamic conditions. As used herein, the term arterial tone represents the combined effect of vascular compliance (i.e., the compliance of the elasticity of the larger vessels) and peripheral resistance (i.e., the resistance to flow of the small peripheral vessels). Similarly, as used herein the phrases hyperdynamic and vasodilation mean a condition in which the peripheral arterial pressure and flow are decoupled from the central aortic pressure and flow, and the term peripheral arteries is intended to mean arteries located away from the heart, e.g., radial, femoral, or brachial arteries. Decoupled arterial pressure means that the normal relationship between peripheral arterial, and central pressure (i.e., the arterial pressure is amplified towards the periphery) is not valid. This also includes conditions in which the peripheral arterial pressure is not proportional or is not a function of the central aortic pressure. Under normal hemodynamic conditions, blood pressure increases the further away from the heart the measurement is taken. Such a pressure increase is shown in Fig. 1, *i.e.,* the amplitude of a pressure wave measured at radial arteries is greater than the pressure measured at the femoral artery, which in turn is greater than the aortic pressure. These differences in pressure are related to wave reflection, *i.e.,* pressure is amplified toward the periphery.

This normal hemodynamic relationship of pressures, *i.e.,* an increase in pressure away from the heart, is often relied upon in medical diagnosis. However, under hyperdynamic/ vasodilation conditions, this relationship can become inverted with the arterial pressure becoming lower than the central aortic pressure. This reversal has been attributed, for example, to arterial tone in the peripheral vessels, which is suggested to impact the wave reflections discussed above. Such a hyperdynamic condition is shown in Fig. 2, *i.e.,* the amplitude of a pressure wave measured at radial arteries is lower than the pressure measured as the femoral artery, which in turn is lower than the aortic pressure. Drugs that dilate small peripheral arteries (*e.g.*, nitrates, ACE inhibitors, and calcium inhibitors) are thought to contribute to hyperdynamic conditions. These types of severe vasodilatory conditions are also often observed in situations right after cardiopulmonary bypass (coronary bypass), in which the radial arterial pressure underestimates the pressure in the aorta. Substantial central to peripheral pressure differences, where the peripheral arterial pressure underestimates the central aortic pressure, are usually observed in patients with severe sepsis who are treated with large amount of fluids and high-dose vasopressors, leading to severe vasodilation. Very similar conditions are also observed in patients with end stage liver disease. As will be well appreciated by those of skill in the art, certain treatments for subjects in normal hemodynamic conditions will be approached differently than for subjects in hyperdynamic conditions. Thus, the presently disclosed methods detect a vascular condition in a subject, if present, and use an appropriate calculation to determine accurate cardiovascular parameters, such as arterial tone and cardiac output, for the subject.

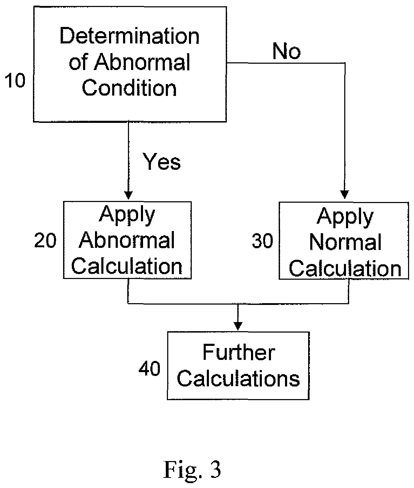

The methods for measuring a cardiovascular parameter in subjects with normal or abnormal hemodynamic conditions as described herein generally include the step of providing arterial pressure waveform data from a subject then steps in which the data are analyzed. (As used herein the term arterial pressure waveform data is intended to mean arterial pressure waveform data or any other signal proportional to, derived from, or a function of the arterial pressure signal.) The result(s) of each analysis step determine the next analysis to be performed. One example of the steps of the methods are shown in Fig. 3. First the arterial pressure waveform is analyzed to determine if the abnormal condition is present (as shown at 10). If the subject is determined to be experiencing the abnormal hemodynamic condition, then a calculation is performed using the arterial pressure waveform data to determine a cardiovascular parameter for abnormal conditions (as shown at 20). If the subject is determined to be experiencing a normal hemodynamic condition, then a calculation is performed on the arterial pressure waveform to determine a cardiovascular parameter for normal hemodynamic conditions (as shown at 30). The appropriate value can then be used to calculate other values related to the cardiovascular parameter, e.g., arterial tone, as needed (as shown at 40).

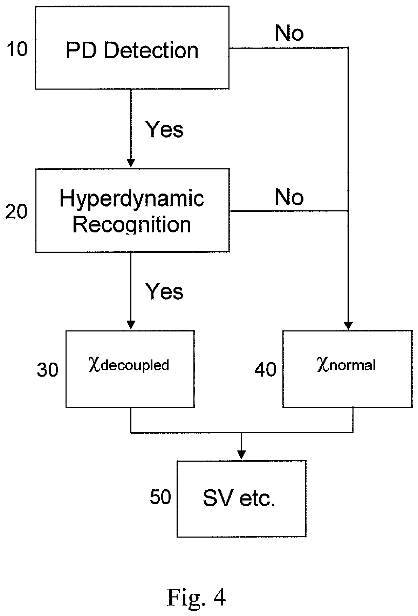

A further example of the steps of a particular method as described herein is shown in Fig. 4. This method involves measuring arterial tone in subjects experiencing hyperdynamic hemodynamic conditions and subjects experiencing normal hemodynamic conditions. In this method, first the arterial pressure waveform is analyzed to determine if the subject's peripheral arterial pressure is decoupled from the subject's central aortic pressure (as shown at 10). If the subject's peripheral arterial pressure is determined to be decoupled from the subject's central aortic pressure, then the arterial pressure waveform data is analyzed to determine if the subject is hyperdynamic (as shown at 20). If the subject also is determined to be hyperdynamic, then a multivariate statistical model is applied to the subject's arterial pressure waveform data to determine the subject's hyperdynamic arterial tone ($\chi_{decoupled}$) (as shown at 30). If the subject's peripheral arterial pressure is not determined to be decoupled from the subject's central aortic pressure or the subject is not determined to be hyperdynamic, then multivariate statistical model is applied to the subject's arterial pressure waveform data to determine the subject's normal arterial tone ($\chi_{normal}$) (as shown at 40). The subject's arterial tone appropriate to their condition (i.e., $c_{normal}$ or $c_{decoupled}$) can then be used to calculate accurate stroke volume, cardiac output, or other values related to arterial tone (as shown at 50). The same method can be used for other cardiovascular parameters.

In these methods, determining if the subject is experiencing normal or abnormal hemodynamic conditions involves applying a multivariate statistical (i.e., Boolean) model to the arterial pressure waveform. Such a multivariate statistical model is prepared from a first set of arterial pressure waveform data from a first group of test or reference subjects that were experiencing the abnormal hemodynamic condition and a second set of arterial pressure waveform data from a second group of test or reference subjects that were experiencing normal hemodynamic conditions. The multivariate Boolean model provides an output value that corresponds to (1) a first established value for the arterial pressure waveforms of the first set of arterial pressure waveform data and (2) a second established value for the arterial pressure waveform of the second set of arterial pressure waveform data. If the output value is greater than a threshold value between the first established value and second established value, then the subject is determined to be experiencing the abnormal hemodynamic condition. Stated another way, this multivariate Boolean model is set up to provide different output values for each set of input data. Specifically, the multivariate Boolean model provides an output value that corresponds to the first established value for the arterial pressure waveforms of the first set of arterial pressure waveform data and the second established value for the arterial pressure waveform of the second set of arterial pressure waveform data.

As a specific example, determining if a subject's peripheral arterial pressure is decoupled from the subject's central aortic pressure involves applying a multivariate statistical (i.e., Boolean) model to the arterial pressure waveform data to determine if the subject's peripheral arterial pressure is decoupled from the subject's central aortic pressure. This multivariate Boolean model is prepared from a first set of arterial pressure waveform data from a first group of test subjects that were experiencing decoupling between peripheral arterial pressure and central aortic pressure and a second set of arterial pressure waveform data from a second group of test subjects that were not experiencing decoupling between peripheral arterial pressure and central aortic pressure. The multivariate Boolean model provides an output value ("decoupled output value") that corresponds to a first decoupled value for the arterial pressure waveforms of the first set of arterial pressure waveform data and a second decoupled value for the arterial pressure waveform of the second set of arterial pressure waveform data. If the decoupled output value is greater than a decoupled threshold value between the first decoupled value and second decoupled value, then the subject's peripheral arterial pressure is determined to be decoupled from the subject's central aortic pressure.

The multivariate Boolean models used herein are based on sets of factors including one or more parameters affected by the abnormal hemodynamic condition. Without wishing to be bound by theory, using one model and constraining the model to different output requirements for the first and second sets of data takes advantage of multiple factors to provide an indication that a vascular condition is occurring in a subject. Each type of factor used, e.g., pulse beats standard deviation, typically registers a difference between subjects experiencing a particular vascular condition and those not experiencing the condition. This difference, however, is often located along a continuum and a particular subject may have a value between a definite positive indication and a definite negative indication or for some reason in that subject the particular factor may appear to be within a normal range even though the subject is experiencing the vascular condition. However, by using multiple factors, i.e., multiple factors impacted by the vascular condition, there will typically be enough positive indications to indicate that a condition is present (or enough negative indications to indicate the condition is not present). Multivariate Boolean models as described herein provide the ability to use multiple factors to increase the ability to differentiate between two states, i.e., experiencing or not experiencing peripheral decoupling.

The specific number of factors used in a multivariate Boolean model will depend on the ability of the individual factors to differentiate between a subject who is experiencing a particular condition and a subject who is not experiencing the particular condition. The number of factors can also be increased to provide a greater level of accuracy to a model. Thus, greater numbers of factors can be used to aid in the precision, accuracy, and/or reproducibility of a model as needed in particular circumstances. Examples of factors that can be used in the models described herein include (a) a parameter based on the pulse beats standard deviation of a set of arterial pressure waveform data, (b) a parameter based on the R-to-R interval of a set of arterial pressure waveform data (inversely proportional to heart rate), (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, and (m) a parameter based on the pressure dependent Windkessel compliance of a set of arterial pressure waveform data. Additional factors that can be used with the multivariate statistical models described herein include (n) a parameter based on the shape of the beat-to-beat arterial blood pressure signal (e.g., time domain, frequency domain, or time-frequency domain measures) and at least one statistical moment of the arterial blood pressure signal having an order of one or greater, (o) a parameter corresponding to the heart rate, and (p) a set of anthropometric parameters of the subject. One or more of these factors (or all of these factors) can be used in the multivariate statistical models described herein.

The factors used in the multivariate Boolean and multivariate statistical models described herein are calculated from signals based on arterial blood pressure or signals proportional to, derived from, or a function of arterial blood pressure. The calculation of cardiovascular parameters, such as arterial compliance (arterial tone), is described in U.S. Patent Application Serial No. 10/890,887, filed July 14, 2004. Example of factors and data used in calculating the cardiovascular parameters for use with the methods disclosed herein, including the parameters discussed in U.S. Patent Application Serial No. 10/890,887, are described below.

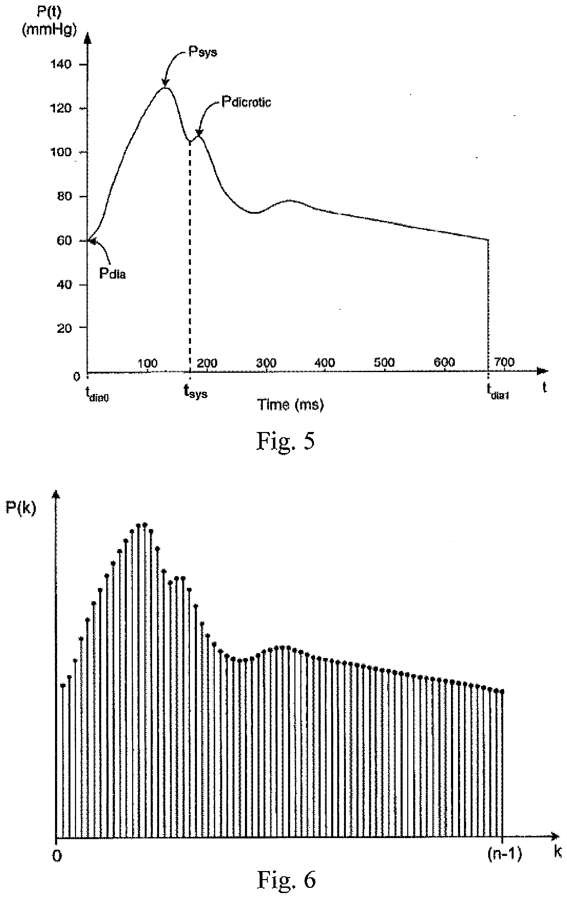

Fig. 5 is an example of an arterial pressure waveform, P(t), taken over a single heart cycle. This heart cycle starts at the point of diastolic pressure $P_{dia}$ at time $t_{dia0}$, through the time $t_{sys}$ of up to systolic pressure $P_{sys}$, to a time $t_{dial}$ at which the blood pressure once again reaches $P_{dia}$.

Signals useful with the present methods include cardiovascular parameters based on arterial blood pressure or any signal that is proportional to, derived from, or a function of arterial blood pressure, measured at any point in the arterial tree, e.g., radial, femoral, or brachial, either invasively or non-invasively. If invasive instruments are used, in particular, catheter-mounted pressure transducers, then any artery is a possible measurement point. Placement of non-invasive transducers will typically be dictated by the instruments themselves, e.g., finger cuffs, upper arm pressure cuffs, and earlobe clamps. Regardless of the specific instrument used, the data obtained will ultimately yield an electric signal corresponding (for example, proportional) to arterial blood pressure.

As illustrated in Fig. 6, analog signals such as arterial blood pressure can be digitized into a sequence of digital values using any standard analog-to-digital converter (ADC). In other words, arterial blood pressure, $t_0 \leq t \leq t_f$, can be converted, using known methods and circuitry, into the digital form P(k), k=0, (n-1), where $t_0$ and $t_f$ are initial and final times of the measurement interval and n is the number of samples of arterial blood pressure to be included in the calculations, distributed usually evenly over the measurement interval.

To capture relevant data from such digital or digitized signals, consider an ordered collection of m values, that is, a sequence Y(i), where i=1,. .., (m-1). As is well known from the field of statistics, the first four moments $\mu_1$, $\mu_2$, $\mu_3$, and $\mu_4$ ofY(i) can be calculated using known formulas, where $\mu_1$ is the mean (*i.e.,* arithmetic average), $\mu_2=\sigma^2$ is the variation (*i.e.,* the square of the standard deviation $\sigma$), $\mu_3$ is the skewness, and $\mu_4$ is the kurtosis. Thus:

$$\mu1=Y_{avg}=1/m*\Sigma(Y(i)) \qquad \text{(Formula 1)}$$

$$\mu_2=\sigma^2=1/(m\text{-}1)*\Sigma(Y(i)\text{-}Y_{avg})^2 \qquad \text{(Formula 2)}$$

$$\mu_3=1/(m\text{-}1)*\Sigma[(Y(i)\text{-}Y_{avg})/\sigma]^3 \qquad \text{(Formula 3)}$$

$$\mu_4=\sigma/(m\text{-}1)*\Sigma[(Y(i)\text{-}Y_{avg})/\sigma]^4 \qquad \text{(Formula 4)}$$

In general, the β-th moment $\mu_p$ can be expressed as:

$$\mu_\beta=1(m\text{-}1)*1/\sigma^\beta*\Sigma[(Y)(i)\text{-}Y_{avg})]^\beta \qquad \text{(Formula 5)}$$

where i=0, ... , (m-1). The discrete-value formulas for the second through fourth moments usually scale by 1/(m-1) instead of 1/m for well-known statistical reasons.

The methods described herein may utilize factors that are a function not only of the four moments of the pressure waveform P(k), but also of a pressure-weighted time vector. Standard deviation $\sigma$ provides one level of shape information in that the greater $\sigma$ is, the more "spread out" the function Y(i) is, *i.e.,* the more it tends to deviate from the mean. Although the standard deviation provides some shape information, its shortcoming can be easily understood by considering the following: the mean and standard deviation will not change if the order in which the values making up the sequence Y(i) is "reversed," that is, Y(i) is reflected about the i=0 axis and shifted so that the value Y(m-1) becomes the first value in time.

Skewness is a measure of lack of symmetry and indicates whether the left or right side of the function Y(i), relative to the statistical mode, is heavier than the other. A positively skewed function rises rapidly, reaches its peak, then falls slowly. The opposite would be true for a negatively skewed function. The point is that the skewness value includes shape information not found in the mean or standard deviation values-in particular, it indicates how rapidly the function initially rises to its peak and then how slowly it decays. Two different functions may have the same mean and standard deviation, but they will then only rarely have the same skewness.

Kurtosis is a measure of whether the function Y(i) is more peaked or flatter than a normal distribution. Thus, a high kurtosis value will indicate a distinct peak near the mean, with a drop thereafter, followed by a heavy "tail." A low kurtosis value will tend to indicate that the function is relatively flat in the region of its peak. A normal distribution has a kurtosis of 3.0; actual kurtosis values are therefore often adjusted by 3.0 so that the values are instead relative to the origin.

An advantage of using the four statistical moments of the beat-to-beat arterial pressure waveform is that the moments are accurate and sensitive mathematical measures of the shape of the beat-to-beat arterial pressure waveform. As arterial compliance and peripheral resistance directly affect the shape of the arterial pressure waveform, the effect of arterial compliance and peripheral resistance could be directly assessed by measuring the shape of the beat-to-beat arterial pressure waveform. The shape sensitive statistical moments of the beat-to-beat arterial pressure waveform along with other arterial pressure parameters described herein could be effectively used to measure the combined effect of vascular compliance and peripheral resistance, *i.e.,* the arterial tone. The arterial tone represents the combined effect of arterial compliance and peripheral resistance and corresponds to the impedance of the well known 2-element electrical analog equivalent model of the Windkessel hemodynamic model, consisting of a capacitive and a resistive component. By measuring arterial tone, several other parameters that are based on arterial tone, such as arterial elasticity, stroke volume, and cardiac output, also could be directly measured. Any of those parameters could be used as factors in the methods described herein.

When the first four moments $\mu_{1P}$, $\mu_{2P}$, $\mu_{3P}$, and $\mu_{4P}$ of the pressure waveform P(k) are calculated and used in a multivariate Boolean or multivariate statistical model, where $\mu_{1P}$ is the mean, $\mu_{2P}$ P=$\sigma_P^2$ is the variation, that is, the square of the standard deviation $\sigma_P$; $\mu_{3P}$ is the skewness, and $\mu_{4P}$ is the kurtosis, where all of these moments are based on the pressure waveform P(k). Formulas 1-4 above may be used to calculate these values after substituting P for Y, k for i, and n for *m.*

Formula 2 above provides the "textbook" method for computing a standard deviation. Other, more approximate methods may also be used. For example, at least in the context of blood pressure-based measurements, a rough approximation to $\sigma_P$ is to divide by three the difference between the maximum and minimum measured pressure values, and that the maximum or absolute value of the minimum of the first derivative of the P(t) with respect to time is generally proportional to $\sigma_P$.

As Fig. 6 illustrates, at each discrete time k, the corresponding measured pressure will be P(k). The values k and P(k) can be formed into a sequence T(j) that corresponds to a histogram, meaning that each P(k) value is used as a "count" of the corresponding k value. By way of a greatly simplified example, assume that the entire pressure waveform consists of only four measured values P(1)=25, P(2)=50, P(3)=55, and P(4)=35. This could then be represented as a sequence T(j) with 25 ones, 50 twos, 55 threes, and 35 fours:
T(j)=1, 1, ... ,1, 2, 2,..., 2, 3, 3, ..., 3, 4, 4, ...,4 This sequence would thus have 25+50+55+35=165 terms.

Moments may be computed for this sequence just as for any other. For example, the mean (first moment) is:

$$\mu_{1T}=(1*25+2*50+3*55+4*35)/165=430/165=2.606 \qquad \text{(Formula 6)}$$

and the standard deviation $\sigma_T$ is the square root of the variation $\mu_{2T}$:

$$\text{SQRT}[1/164*25(1-2.61)^2+50(2-2.61)^2+55(3-2.61)^2+35(4-2.61)^2]=0.985$$

The skewness $\mu_{3T}$ and kurtosis $\mu_{4T}$ can be computed by similar substitutions in Formulas 3 and 4:

$$\mu_{3T}=\{1/(164)*(1/\sigma_T^3)\Sigma[P(k)*(k-\mu_{1T})^3]\} \qquad \text{(Formula 7)}$$

$$\mu_{4T}=\{1/(164)*(1/\sigma_T^4)\Sigma[P(k)*(k-\mu_{1T})^4]\} \qquad \text{(Formula 8)}$$

where k=1, ... , (m-1).

As these formulas indicate, this process in effect "weights" each discrete time value k by its corresponding pressure value P(k) before calculating the moments of time. The sequence T(j) has the very useful property that it robustly characterizes the timing distribution of the pressure waveform. Reversing the order of the pressure values P(k) will in almost all cases cause even the mean of T(j) to change, as well as all of the higher-order moments. Moreover, the secondary "hump" that normally occurs at the dicrotic pressure $P_{dicrotic}$ also noticeably affects the value of kurtosis $\mu_{4T}$; in contrast, simply identifying the dicrotic notch in the prior art, such as in the Romano method, requires noisy calculation of at least one derivative.

The pressure weighted moments provide another level of shape information for the beat-to-beat arterial pressure signal, as they are very accurate measures of both the amplitude and the time information of the beat-to-beat arterial pressure signal. Use of the pressure weighted moments in addition to the pressure waveform moments can increase the accuracy of the models described herein.

One cardiovascular parameter useful with the methods described herein is the arterial tone factor $\chi$, which can be used as a cardiovascular parameter by itself or in the calculation of other cardiovascular parameters such as stroke volume or cardiac output. Calculation of the arterial tone $\chi$ uses all four of the pressure waveform and pressure-weighted time moments. Additional values are included in the computation to take other known characteristics into account, *e.g.*, patient-specific complex pattern of vascular branching. Examples of additional values include, heart rate HR (or period of R-waves), body surface area BSA, or other anthropometric parameters of the subject, a compliance value C(P) calculated using a known method such as described by Langewouters et al. ("The Static Elastic Properties of 45 Human Thoracic and 20 Abnormal Aortas in vitro and the Parameters of a New Model," J. Biomechanics, 17(6):425-435 (1984)), which computes compliance as a polynomial function of the pressure waveform and the patient's age and sex, a parameter based on the shape of the arterial blood pressure signal and at least one statistical moment of the arterial blood pressure signal having an order of one or greater, a parameter based on the area under the systolic portion of the arterial blood pressure signal, a parameter based on the duration of the systole, and a parameter based on the ratio of the duration of the systole to the duration of the diastole.

These last three cardiovascular parameters, *i.e.,* the area under the systolic portion of the arterial blood pressure signal, the duration of the systole, and the ratio of the duration of the systole to the duration of the diastole, are impacted by arterial tone and vascular compliance and, thus, vary, for example, between subjects in normal hemodynamic conditions and subjects in hyperdynamic conditions. Because these three cardiovascular parameters vary between normal and hyperdynamic subjects the methods described herein can use these cardiovascular parameters to detect vasodilation or vasoconstriction in the peripheral arteries of a subject.

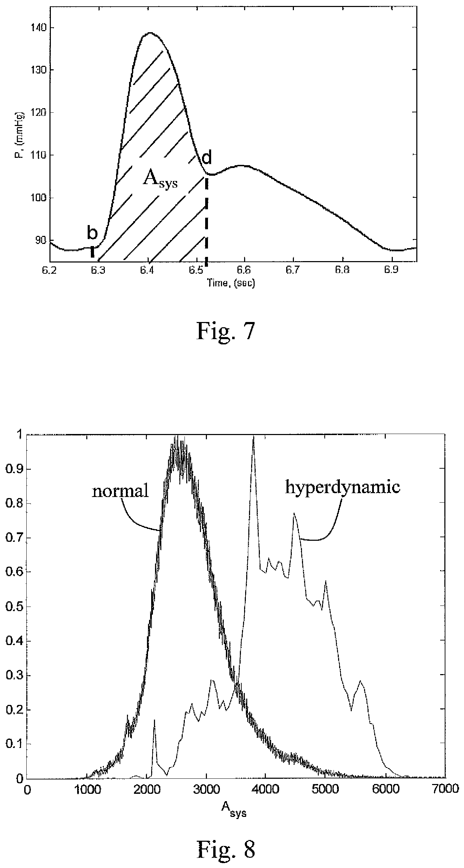

The area under the systolic portion of an arterial pressure waveform ($A_{sys}$) is shown graphically in Fig. 7. The area under the systolic portion of the arterial pressure waveform in an arterial pressure signal is defined as the area under the portion of the waveform starting from the beginning of the beat and ending in the dichrotic notch (from point b to point d on Fig. 7). The area under the systole represents the energy of the arterial pressure signal during systole, which is directly proportional to stroke volume and inversely proportional to arterial compliance. When measured over groups of normal and hyperdynamic patients a shift in $A_{sys}$ can be detected. As shown in Fig. 8, the energy of the arterial pressure signal during systole is higher, for example, in some subjects in hyperdynamic conditions. Those subjects with higher $A_{sys}$ are typically subjects with high cardiac output (CO) and low or normal HR, where the elevated CO is mainly caused by elevated heart contractility, which means that those subjects have increased stroke volume and decreased arterial compliance, which is directly reflected in the energy of the arterial pressure signal during systole. The reflected waves, which are usually very intense during many hyperdynamic conditions, may have also significant contribution to the increased energy of the signal during systole.

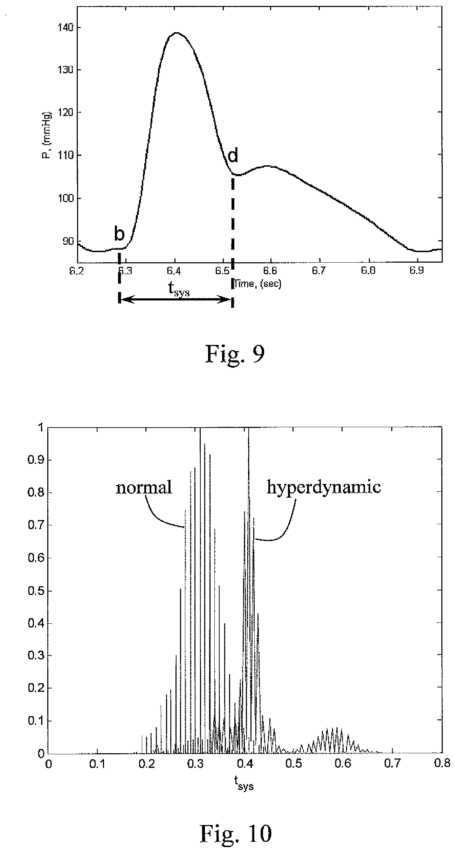

The duration of the systole ($t_{sys}$) is shown graphically in Fig. 9. The duration of the systole in an arterial pressure waveform is defined as the time duration from the beginning of the beat to the dichrotic notch (from point b to point d on Fig. 9). The duration of the systole is directly affected by the arterial compliance and is relatively independent of the changes in peripheral arterial tone, except when large reflect waves are present. As shown on Fig. 10, for example, the duration of the systole in some hyperdynamic subjects is higher than the duration of the systole in normal subjects (data shifted toward higher $t_{sys}$ values). As seen for the systolic energy, the duration of the systole is typically higher in patients with high CO who also have low or normal HR, where the elevated CO is mainly caused by elevated heart contractility and where the contractility may not have been high enough to increase the systolic energy. The increased stroke volume in those patients is partially due to increased contractility and partially due to increased duration of the systole. Reflected waves play a role here as well.

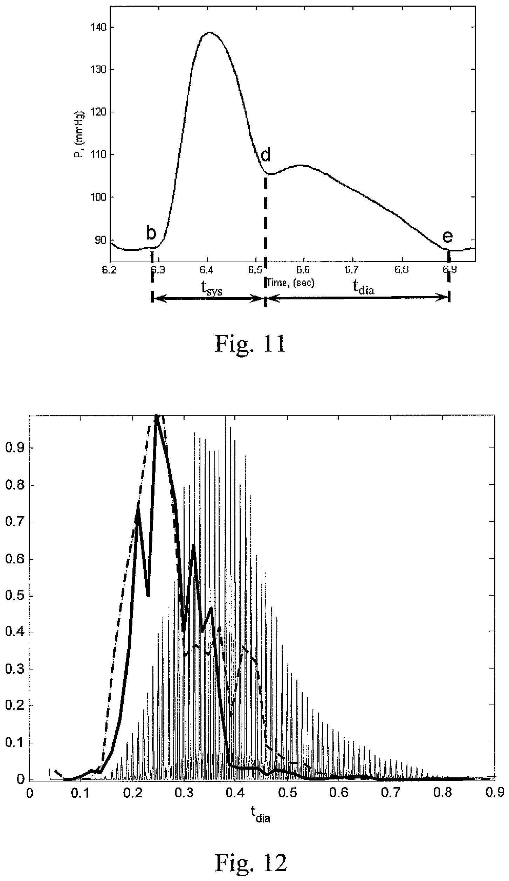

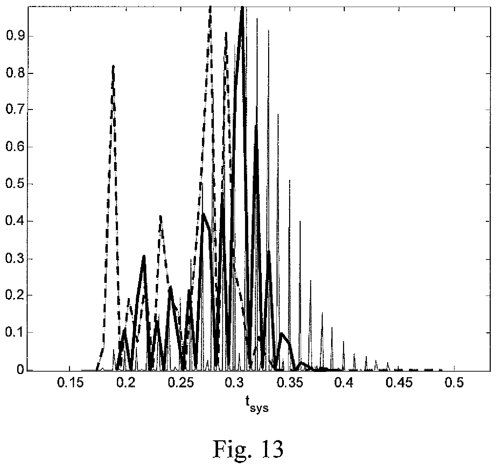

A further parameter that varies, for example, between normal and hyperdynamic subjects is the ratio of the duration of the systole ($t_{sys}$) and the duration of the diastole ($t_{dia}$), as shown graphically in Fig. 11. The duration of the diastole in an arterial pressure waveform is defined as the time duration from the dichrotic notch to the end of the cardiac cycle (from point d to point e on Fig. 11). In some hyperdynamic conditions, the ratio of the durations of the systole and diastole is significantly higher than that observed in normal hemodynamic conditions. This is typically observed in septic shock patients with elevated CO where HR is also high. In these types of conditions, the systole takes over almost the entire cardiac cycle leaving very little time for the diastole before the next cardiac cycle begins. This is shown in Figs. 12 and 13, which show the duration of diastole (Fig. 12) and the duration of systole (Fig. 13) during high HR conditions in septic shock patients and in normal patients. As shown in the figures, high HR patients in normal hemodynamic conditions (dashed line) tend to have low durations of both the systole and the diastole, while high HR patients in septic shock (thick line) tend to have low duration of the diastole but normal or high duration of the systole.

Other parameters based on the arterial tone factor such as, for example, Stroke Volume (SV), Cardiac Output (CO), Arterial Flow, or Arterial Elasticity can be used as factors in the methods described herein. As an example, Stroke Volume (SV) can be calculated as the product of the arterial tone and the standard deviation of the arterial pressure signal:

$$SV = \chi \cdot \sigma_P \qquad \text{(Formula 9)}$$

where:

SV is stroke volume;
$\chi$ is arterial tone; and
$\sigma_p$ is the standard deviation of the arterial pressure

The analog measurement interval, that is, the time window [$t_0$, $t_f$], and thus the discrete sampling interval k=0, ... , (n-1), over which each calculation period is conducted should be small enough so that it does not encompass substantial shifts in the pressure and/or time moments. However, a time window extending longer than one cardiac cycle will provide suitable data. Preferably, the measurement interval is a plurality of cardiac cycles that begin and end at the same point in different cardiac cycles. Using a plurality of cardiac cycles ensures that the mean pressure value used in the calculations of the various higher-order moments will use a mean pressure value $P_{avg}$ that is not biased because of incomplete measurement of a cycle.

Larger sampling windows have the advantage that the effect of perturbations such as those caused by reflections are typically reduced. An appropriate time window can be determined using normal experimental and clinical methods well known to those of skill in the art. Note that it is possible for the time window to coincide with a single heart cycle, in which case mean pressure shifts will not be of concern.

The time window $[t_0, t_f]$ is also adjustable according to drift in $P_{avg}$. For example, if $P_{avg}$ over a given time window differs absolutely or proportionately by more than a threshold amount from the $P_{avg}$ of the previous time window, then the time window can be reduced; in this case stability of $P_{avg}$ is then used to indicate that the time window can be expanded. The time window can also be expanded and contracted based on noise sources, or on a measure of signal-to-noise ratio or variation. Limits are preferably placed on how much the time window is allowed to expand or contract and if such expansion or contraction is allowed at all, then an indication of the time interval is preferably displayed to the user.

The time window does not need to start at any particular point in the cardiac cycle. Thus, $t_0$ need not be the same as $t_{dia0}$, although this may be a convenient choice in many implementations. Thus, the beginning and end of each measurement interval (*i.e.,* $t_0$ and $t_f$) may be triggered on almost any characteristic of the cardiac cycle, such as at times $t_{dia0}$ or $t_{sys}$, or on non-pressure characteristics such as R waves, etc.

Rather than measure blood pressure directly, any other input signal may be used that is proportional to, derived from, or a function of blood pressure. This means that calibration may be done at any or all of several points in the calculations. For example, if a signal other than arterial blood pressure itself is used as input, then it may be calibrated to blood pressure before its values are used to calculate the various component moments, or afterwards, in which case either the resulting moment values can be scaled. In short, the fact that the cardiovascular parameter may in some cases use a different input signal than a direct measurement of arterial blood pressure does not preclude its ability to generate an accurate compliance estimate.

In one example, the decoupled output value for a multivariate Boolean model for a given set of factors is constrained to a first decoupled value for those factors as obtained from the arterial pressure waveforms of the first set of arterial pressure waveform data and at the same time constrained to a second decoupled value for those factors as obtained from the arterial pressure waveform of the second set of arterial pressure waveform data. The first decoupled value and second decoupled value can be set to provide a convenient comparison to decoupled output values for a subject being analyzed. For example, the first decoupled value can be greater than the second decoupled value. Additionally, the first decoupled value can be a positive number and the second decoupled value can be a negative number. For further example, the first decoupled value can be +100 and the second decoupled value can be -100.

Such decoupled values, i.e., +100 and -100, can be used to create a Boolean-type outcome for the multivariate Boolean model. For example, for a multivariate Boolean model with a first decoupled value of +100 and a second decoupled value of -100, the decoupled output value could be set up with the following indicators:

Decoupled Output $\geq 0 \rightarrow$ vascular condition indicated

Decoupled Output $< 0 \rightarrow$ vascular condition not indicated

In this case the "0" value can be considered a threshold value at or above which a decoupled condition is indicated, i.e., values greater than or equal to zero more closely relate to the subjects used to establish the multivariate model that were experiencing decoupling than to those subjects that were not experiencing decoupling. Conversely, values lower than "0" indicate that the subject is experiencing decoupling more closely related to those subjects used to establish the multivariate model that were not experiencing decoupling than to those subjects that were experiencing decoupling. For this example, the threshold value was set at the mean between the first decoupled value and the second decoupled value. The threshold value could, however, be shifted based upon empirical observations. For example, if a value $\alpha$ is the mean value between the first and second threshold values, then the threshold value could be $\alpha$-1, $\alpha$-2, $\alpha$-3, $\alpha$ -4, $\alpha$ -5, $\alpha$ -10, $\alpha$ -15, $\alpha$ -20, $\alpha$ +1, $\alpha$ +2, $\alpha$ +3, $\alpha$ +4, $\alpha$ +5, $\alpha$ +10, $\alpha$ +15, or $\alpha$ +20.

In multivariate Boolean models as described herein there may be a range of threshold values that are not determinative of whether a subject is experiencing decoupling or another vascular condition. In these cases, a threshold range can be used. For example, for a multivariate Boolean model with a first decoupled value of +100 and a second decoupled value of -100, the model output could be set up with the following indicators:

Decoupled Output $\geq 10 \rightarrow$ vascular condition indicated

-10 < Decoupled Output < 10 → indeterminate (continue to analyze)

Decoupled Output ≤ -10 → vascular condition not indicated

In this example, values between -10 and 10 are considered indeterminate and further data can be analyzed to see if a value greater than or equal to 10 or less than or equal to -10 is indicated. Otherwise, for this situation, decoupled output values greater than or equal to 10 indicate decoupling and values less than or equal to -10 indicate a normal vascular condition. The threshold range will depend upon an evaluation of the ability of the model to indicate a vascular condition at the intermediate point between the first and second decoupled values. Additionally, the threshold range can be shifted or otherwise adjusted based upon empirical observations. For example, if a value $\alpha$ is the mean value between the first and second threshold values, then the threshold value could be $[(\alpha-1)\pm\beta]$, $[(\alpha-2)\pm\beta]$, $[(\alpha-3)\pm\beta]$, $[(\alpha-4)\pm\beta]$, $[(\alpha-5)\pm\beta]$, $[(\alpha-10)\pm\beta]$, $[(\alpha-15)\pm\beta]$, $[(\alpha-20)+\beta]$, $[(\alpha+1)\pm\beta]$, $[(\alpha+2)\pm\beta]$, $[(\alpha+3)\pm\beta]$, $[(\alpha+4)\pm\beta]$, $[(\alpha+5)\pm\beta]$, $[(\alpha+10)\pm\beta]$, $[(\alpha+15)\pm\beta]$, or $[(\alpha+20)\pm\beta]$, where $\beta$ is the upper and lower bounds of the range, e.g., $\beta$ can be 1, 2, 3, 4, 5, 6, 7, 8, 9, 10, 15, or 20 depending on the model.

Creating a multivariable Boolean model involves several steps. For example, a multiple linear regression response surface can be used to establish the model. The number of terms used in the model can be determined using several numerical approaches to minimize the mean square error between the model output value and the values the model is forced to for the specific conditions. As a more detailed example, a predictor variable set for the model output value is related to a specific patient group, i.e., the established value can be set to +100 for subjects experiencing a specific vascular condition and can be set to -100 for subjects not experiencing the vascular condition. This operation creates a suite of reference values, each of which is a function of the component parameters of the model output value. A multivariate approximating function can then be computed using known numerical methods that best relate the model outputs to either +100 or -100 depending on the subject group in the defined manner. A polynomial multivariate fitting function can then be used to generate the coefficients of the polynomial that satisfies the +100 and -100 constraints for each set of predictor values. Such a multivariate model (where $\beta$ is model output) has the following general form:

$$\beta = \begin{bmatrix} a_1 & a_2 & ... & a_n \end{bmatrix} * \begin{bmatrix} x_1 \\ x_2 \\ ... \\ x_n \end{bmatrix} \text{ (Formula 10)}$$

Where $a_1 ... a_n$ are the coefficients of the polynomial multi-regression model, and $x_1 ... x_n$ are the model's predictor variables. The predictor variables are selected from the factors discussed above that are derived from the arterial pressure waveforms.

Each of the model's predictor variables $x_i$ is a predefined combination of the arterial pressure waveform parameters $v_i$ and can be computed as follows:

$$x_i = \prod_m \left( \begin{bmatrix} v_1 & v_2 & ... & v_m \end{bmatrix}^{\wedge \begin{bmatrix} P_{1,1} & ... & P_{1,m} \\ ... & ... & ... \\ P_{n,1} & ... & P_{n,m} \end{bmatrix}} \right) \text{ (Formula 11)}$$

The coefficients $a_i$ and the exponent matrix "P" can be determined by multivariate least-squares regression using the factor data collected from the subjects. They can be related, for example, to the +100 and -100 values depending on the patient's group for the population of reference subjects. For example, factor data from subjects experiencing decoupling can be related as follows:

$$\beta = \begin{bmatrix} a_1 & a_2 & \dots & a_n \end{bmatrix} * \begin{bmatrix} x_1 \\ x_2 \\ \dots \\ x_n \end{bmatrix} = +100 \qquad \text{(Formula 12)}$$

And factor data from subjects not experiencing decoupling can be related as follows:

$$\beta = \begin{bmatrix} a_1 & a_2 & \dots & a_n \end{bmatrix} * \begin{bmatrix} x_1 \\ x_2 \\ \dots \\ x_n \end{bmatrix} = -100 \qquad \text{(Formula 13)}$$

As a specific example, a multivariate Boolean model was created using 14 arterial pressure waveform factors ($X_i$) in which 17 terms were required to provide a best fit for the model. These parameters were: $v_1$ (pulse beats standard deviation (std)), $v_2$ (R-to-R interval (r2r)), $v_3$ (area under the systole (sys area)), $v_4$ (the duration of the systole (t_sys)), $v_5$ (the duration of the diasystole (t_dia)), $v_6$ (mean arterial pressure (MAP)), $v_7$ (pressure weighted standard deviation ($\sigma_T$)), $v_8$ (pressure weighted mean ($\mu_2$)), $v_9$ (skewness of the arterial pulse beats ($\mu_{3P}$)), $v_{10}$ (kurtosis of the arterial pulse pressure ($\mu_{4P}$)), $v_{11}$ (pressure weighted skewness ($\mu_{3T}$)), $v_{12}$ (pressure weighted kurtosis ($\mu_{4T}$))) $v_{13}$ (pressure dependent Windkessel compliance ($C_W$)), and $v_{14}$ (patient body surface area (BSA)). The model was as follows:

$$X_i = \prod_{14} \left( \begin{bmatrix} v_1 & v_2 & \dots & v_{14} \end{bmatrix} ^{\wedge \begin{bmatrix} P_{1,1} & \dots & P_{1,14} \\ \dots & \dots & \dots \\ P_{17,1} & \dots & P_{17,14} \end{bmatrix}} \right) \quad \text{(Formula 14)}$$

After regression, the values of the array P (17 × 14) were determined, defining which variables are included in the model as follows:

$$P = \begin{bmatrix}
0 & 0 & 0 & 2 & 0 & 0 & 0 & 0 & 0 & 1 & 0 & 0 & 1 & 0 \\
1 & 0 & -1 & 0 & 0 & 0 & 0 & 0 & 0 & 0 & 0 & 0 & 0 & -1 \\
0 & 0 & 0 & 2 & 0 & 0 & 0 & 0 & 0 & 1 & 0 & 0 & 2 & 0 \\
0 & 0 & 0 & -2 & 0 & 0 & -1 & 0 & 0 & 1 & 0 & 0 & 0 & 0 \\
0 & 0 & 0 & 0 & 0 & 0 & 0 & 0 & 0 & -1 & 0 & 0 & 0 & 2 \\
-1 & 0 & 2 & 0 & 0 & 0 & 0 & 0 & 1 & 0 & 0 & 0 & 0 & 0 \\
1 & 0 & 0 & 0 & 0 & -2 & 0 & 0 & 0 & 0 & 0 & 0 & 2 & 0 \\
0 & 0 & 0 & -1 & 0 & 0 & -2 & 0 & 0 & 0 & 0 & 0 & 0 & 2 \\
0 & 0 & 0 & 0 & 0 & 0 & 0 & 0 & 2 & 0 & 0 & 0 & 1 & 0 \\
0 & 0 & 0 & 0 & 0 & 0 & 0 & 2 & 0 & -2 & 0 & 0 & 2 & 0 \\
0 & 0 & 0 & 1 & 0 & 0 & 0 & 1 & 0 & 0 & 0 & 0 & 2 & 0 \\
0 & 0 & 0 & 0 & 0 & 0 & 0 & 1 & 1 & -1 & 0 & 0 & 0 & 0 \\
0 & 0 & 0 & -2 & 0 & 0 & 0 & 0 & 0 & 2 & 0 & 0 & 0 & 2 \\
0 & 0 & 0 & -1 & 0 & 0 & -2 & 0 & 0 & 1 & 0 & 0 & 0 & 0 \\
0 & 0 & 0 & 0 & 0 & 1 & 0 & 0 & 2 & 0 & 0 & 0 & 0 & 2 \\
0 & 0 & 0 & 0 & 0 & 0 & 1 & 0 & 2 & -2 & 0 & 0 & 0 & 0 \\
2 & 0 & 0 & 0 & 0 & 0 & 0 & 0 & -2 & 0 & 0 & 0 & 0 & 2
\end{bmatrix}$$

The regression was performed such that the number of parameters per regression term was restrained to less than three, with each parameter having an order no greater than two. Thus, as shown above, each row of the matrix P has at most three non-zero terms, with the absolute value of each element of P being at most two. These constraints were set for the sake of numerical stability and accuracy. The expression for β therefore became a 17-term second order curve in 14- dimensional parameter space. The resultant expression for β can be written as:

$$\beta = \begin{bmatrix} a_1 & a_2 & \dots & a_n \end{bmatrix} * \begin{bmatrix} x_1[1] \\ x_1[2] \\ \dots \\ x_1[17] \end{bmatrix} \qquad \text{(Formula 15)}$$

Where $a_1 \dots a_{17}$ are the coefficients of the polynomial multiregression model. These coefficients were then computed (using known numerical methods) to best relate the parameters to β given the chosen suite of factors to equal +100. In this case the chosen factors were determined by formula 14 based on the waveform parameters $v_j$ (defined above). A polynomial multivariate fitting function based on a least-squares regression was used to generate the following coefficients for the polynomial:

$$A = \begin{bmatrix} 0.35578 \\ -1.6207 \\ -0.53381 \\ -28.818 \\ 1.996 \\ 0.41195 \\ 0.60126 \\ -107.64 \\ -1.3753 \\ -0.065631 \\ 0.18202 \\ -0.43511 \\ 0.28365 \\ 265.62 \\ -0.26331 \\ 0.81367 \\ -0.0048077 \end{bmatrix}$$

Once such a multivariate Boolean model is developed, it can be used to detect the decoupled condition of any subject continuously in real-time. The model can be continuously evaluated using the chosen factors determined from a subject's arterial pressure waveform. In this example, the first decoupled value was set at +100 and the second decoupled value was set at - 100, so the threshold value could, for example, be set at zero in this model to provide:

$\beta \geq 0 \rightarrow$ decoupling indicated

$\beta < 0 \rightarrow$ decoupling not indicated

In the present methods, if the subject is determined to be decoupled, then whether the subject is also hyperdynamic is determined. Determining if a subject is hyperdynamic involves applying a hyperdynamic multivariate Boolean model to the arterial pressure waveform data. This hyperdynamic multivariate Boolean model is prepared from a first set of arterial pressure waveform data from a first group of subjects that were hyperdynamic and a second set of arterial pressure waveform data from a second group of subjects that were not hyperdynamic using the same methodology as described above for the decoupled multivariate Boolean model. The hyperdynamic multivariate Boolean model provides a hyperdynamic output value that corresponds to a first hyperdynamic value for the arterial pressure waveforms of the first set of arterial pressure waveform data and a second hyperdynamic value for the arterial pressure waveform of the second set of arterial pressure waveform data. If the hyperdynamic output value is greater than or equal to a hyperdynamic threshold value between the first hyperdynamic established value and second hyperdynamic established value, then the subject is determined to be hyperdynamic. The first and second hyperdynamic values and the hyperdynamic threshold values are determined and/or established in the same manner as the analogous values were established for the decoupled multivariate Boolean model.

As an example of the present methods, if the subject is determined to be decoupled and hyperdynamic, a hyperdynamic multivariate statistical model created using arterial pressure waveform data from a group of test subjects that were experiencing hyperdynamic conditions and decoupling between central and peripheral arterial pressure is used to determine the subject's hyperdynamic and decoupled arterial tone (or other cardiovascular parameter). Similarly, in the present methods, if the subject is not determined to be decoupled or not determined to be hyperdynamic, a normal multivariate statistical model created using arterial pressure waveform data from a group of test subjects that were not experiencing hyperdynamic conditions and not experiencing decoupling between central and peripheral arterial pressure is used to determine the subject's normal arterial tone (or other cardiovascular parameter). The normal and hyperdynamic multivariate statistical models are created using the same methods as described below.

Creating a multivariate statistical model to calculate arterial tone, as an example of a cardiac parameter, is accomplished similarly to creating a multivariate Boolean model as discussed above. The main difference is that the model $\chi$ is not constrained to any established values. Rather, the multivariate model $\chi$ for arterial tone is estimated using known numerical methods that best relates multiple parameters of the arterial pressure waveform of the model $\chi$ to the actual arterial tone (as defined in formula 9 as $SV/\sigma_p$) given the suite of SV and arterial pressure measurements in some predefined sense. Specifically, a polynomial multivariate fitting function is used to generate the coefficients of the polynomial that give a value of $\chi$ for each set of the arterial pressure waveform parameters, as follows:

$$\chi = \begin{bmatrix} a_1 & a_2 & ... & a_n \end{bmatrix} * \begin{bmatrix} x_1 \\ x_2 \\ ... \\ x_n \end{bmatrix} \quad \text{(Formula 16)}$$

Where $a_1 ... a_n$ are the coefficients of the polynomial multi-regression model, and $\chi_1 ... \chi_n$ are the model's predictor variables. The predictor variables are selected from the factors discussed above that are derived from the arterial pressure waveforms.

Each of the model's predictor variables $\chi_i$ is a predefined combination of the arterial pressure waveform parameters $v_i$ and can be computed as follows:

$$x_i = \prod_m \left( \begin{bmatrix} v_1 & v_2 & ... & v_m \end{bmatrix}^{\wedge \begin{bmatrix} P_{1,1} & ... & P_{1,m} \\ ... & ... & ... \\ P_{n,1} & ... & P_{n,m} \end{bmatrix}} \right) \quad \text{(Formula 17)}$$

The coefficients $v_i$ are different time and frequency domain parameters of the arterial pressure waveform.

The model approximation is accomplished using reference measurements from a reference patient's group. The arterial tone model $\chi_{decoupled}$ used for hyperdynamic patients experiencing peripheral arterial pressure decoupling is built using a reference data set including measurements from both normal patients and patients in hyperdynamic conditions experiencing peripheral pressure decoupling. The arterial tone model $\chi_{normal}$ used for both patients in normal hemodynamic conditions is built using a reference data set including measurements from patients in normal conditions.

As a specific example, a multivariate statistical model was created using 11 arterial pressure waveform parameters were used. These parameters were: $v_1$ (standard deviation of the arterial pulse pressure ($\sigma_P$)), $v_2$ (heart rate), $v_3$ (mean arterial pressure ($P_{avg}$)), $v_4$ (pressure weighted standard deviation ($\sigma_T$)), $v_5$ (pressure weighted MAP($\mu_{1T}$)), $v_6$ (skewness of the arterial pulse pressure ($\mu_{3P}$)), $v_7$ (kurtosis of the arterial pulse pressure ($\mu_{4P}$)), $v_8$ (pressure weighted skewness ($\mu_{3T}$)), $v_9$ (pressure weighted kurtosis ($\mu_{4T}$)), $v_{10}$ (pressure dependent Windkessel compliance ($C_W$)), and $v_{11}$ (patient body surface area (BSA)). The coefficients $a_i$ and the exponent matrix "P" can be determined by multivariate least-squares regression using the factor data collected from the subjects. The coefficients and exponent factor are related to the "true" stroke volume, determined through thermodilution, for a population of reference subjects. In this model A and P were established as follows:

*A*=2.95 -0.43472 12.384 -143.49 21.396 -1.3508 0.029824 -7.3862

$$P = \begin{bmatrix} 0 & 0 & 0 & 0 & 0 & 0 & 0 & 0 & 0 & 0 & 1 \\ 0 & 0 & 0 & 0 & 0 & 0 & 0 & 0 & 0 & -1 & 0 \\ 0 & 0 & 2 & 0 & 0 & 0 & 0 & 0 & 0 & 0 & -2 \\ 0 & -2 & 1 & 0 & 0 & 0 & 0 & 0 & 0 & 0 & 0 \\ 0 & -2 & 0 & 0 & 0 & 0 & 0 & 0 & 0 & -1 & 0 \\ 1 & 0 & 0 & 0 & 0 & 0 & -1 & 0 & 0 & 0 & 0 \\ -2 & 0 & 0 & 0 & 0 & 0 & 0 & 0 & 2 & 0 & 0 \\ 0 & -1 & 0 & 0 & 0 & 0 & 0 & 0 & 0 & -1 & -2 \end{bmatrix}$$

Regression was performed in a way to restrain the number of parameters per regression variable to less than three, with each parameter having an order no greater than two. Thus, each row of the matrix P has at most three non-zero terms, with the absolute value of each element of P being at most two. These constraints were used to establish numerical stability and accuracy. The expression for $\chi$ therefore became a second-order curve in eleven-dimensional parameter space. The polynomial expression determined for $\chi$ can be written as follows:

$$x = 2.95 \cdot BSA - 0.43472 \cdot \frac{1}{100 \cdot C_W} + 12.384 \cdot \frac{\left(\frac{1}{100} P_{avg}\right)^2}{BSA^2} - 143.49 \cdot \frac{\frac{1}{100} P_{avg}}{\left(10 \cdot \frac{1}{HR}\right)^2} + 21.396 \cdot$$

$$\frac{1}{100 \cdot C_W \left(10 \cdot \frac{1}{HR}\right)^2} - 1.3508 \cdot \frac{\frac{1}{10} \sigma_P}{\mu_{4P} + 3} + 0.029824 \cdot \frac{(\mu_{4P} + 3)^2}{\left(\frac{1}{10} \sigma_P\right)^2} -$$

$$7.3862 \cdot \frac{1}{10 \cdot \frac{1}{HR} \cdot 100 \cdot C_W \cdot BSA^2}$$

Thus, a subject's cardiovascular parameters can be determined by first creating a model as just described (i.e., determining an approximating function relating a set of clinically derived reference measurements representing blood pressure parameters dependent upon a cardiovascular parameter, the approximating function being a function of one or more of the parameters described above, and a set of clinically determined reference measurements representing blood pressure parameters dependent upon the cardiovascular parameter from subjects with normal hemodynamic conditions or subjects experiencing abnormal hemodynamic conditions (depending on the model)). Next determining a set of arterial blood pressure parameters from the arterial blood pressure waveform data, the set of arterial blood pressure parameters including the same parameters used to create the multivariate statistical model. Then, estimating the subject's cardiovascular parameter by evaluating the approximating function with the set of arterial blood pressure parameters.

These methods for determining a cardiovascular parameter in a subject can be used to continuously calculate a subject's cardiovascular parameter, so that possible changes in condition over time are accounted for. The method can further alert a user when abnormal conditions are determined. Such an alert can be a notice published on a graphical user interface or a sound.

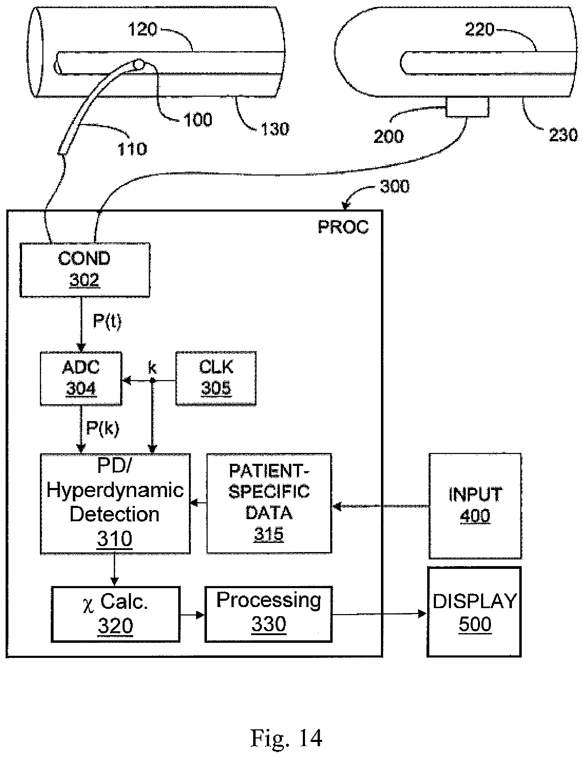

Fig. 14 shows the main components of a system that implements the methods described herein for determining a cardiovascular parameter, such as arterial tone, in a subject. The methods may be implemented within an existing patient-monitoring device, or it may be implemented as a dedicated monitor. As is mentioned above, pressure, or some other input signal proportional to, derived from, or a function of pressure, may be sensed in either or, indeed, both, of two ways: invasively and non-invasively. For convenience, the system is described as measuring arterial blood pressure as opposed to some other input signal that is converted to pressure.

Fig. 14 shows both types of pressure sensing for the sake of completeness. In most practical applications of the methods described herein, either one or several variations will typically be implemented. In invasive applications of the methods described herein, a conventional pressure sensor 100 is mounted on a catheter 110, which is inserted in an artery 120 of a portion 130 of the body of a human or animal patient. The artery 120 is any artery in the arterial system, such as, for example, the femoral, radial or brachial artery. In the non-invasive applications of the methods described herein, a conventional pressure sensor 200, such as a photo-plethysmographic blood pressure probe, is mounted externally in any conventional manner, for example using a cuff around a finger 230 or a transducer mounted on the wrist of the patient. Fig. 14 schematically shows both types.

The signals from the sensors 100, 200 are passed via any known connectors as inputs to a processing system 300, which includes one or more processors and other supporting hardware and system software (not shown) usually included to process signals and execute code. The methods described herein may be implemented using a modified, standard, personal computer, or may be incorporated into a larger, specialized monitoring system. For use with the methods described herein, the processing system 300 also may include, or is connected to, conditioning circuitry 302 which performs normal signal processing tasks such as amplification, filtering, or ranging, as needed. The conditioned, sensed input pressure signal P(t) is then converted to digital form by a conventional analog-to-digital converter ADC 304, which has or takes its time reference from a clock circuit 305. As is well understood, the sampling frequency of the ADC 304 should be chosen with regard to the Nyquist criterion so as to avoid aliasing of the pressure signal (this procedure is very well known in the art of digital signal processing). The output from the ADC 304 will be the discrete pressure signal P(k), whose values may be stored in conventional memory circuitry (not shown).

The values P(k) are passed to or accessed from memory by a software module 310 comprising computer-executable code for implementing the multivariate Boolean models to determine if a subject is peripherally decoupled and/or hyperdynamic. The design of such a software module 310 will be straight forward to one of skill in the art of computer programming.

If used, patient-specific data such as age, height, weight, BSA, etc., is stored in a memory region 315, which may also store other predetermined parameters such as threshold or threshold range values. These values may be entered using any known input device 400 in the conventional manner.

Calculation of arterial tone using the appropriate multivariate statistical model is done in module 320. Calculation module 320 includes computer-executable code and take as inputs the output of module 310, then performs the chosen arterial tone calculations.

As illustrated by Fig. 14, the results may be passed to further modules (330) for additional processing and ultimately displayed on a conventional display or recording device 500 for presentation to and interpretation by a user. As with the input device 400, the display 500 will typically be the same as is used by the processing system for other purposes.

For each of the methods described herein, when decoupling is detected, a user can be notified. The user can be notified of the decoupling by publishing a notice on display 500 or another graphical user interface device. Further, a sound can be used to notify the user of the decoupling. Both visual and auditory signals can be used.

Exemplary embodiments of the present invention have been described above with reference to block diagrams and flowchart illustrations of methods, apparatuses, and computer program products. One of skill will understand that each block of the block diagrams and flowchart illustrations, and combinations of blocks in the block diagrams and flowchart illustrations, respectively, can be implemented by various means including computer program instructions. These computer program instructions may be loaded onto a general purpose computer, special purpose computer, or other programmable data processing apparatus to produce a machine, such that the instructions which execute on the computer or other programmable data processing apparatus create a means for implementing the functions specified in the flowchart block or blocks.

The methods described herein further relate to computer program instructions that may be stored in a computer-readable memory that can direct a computer or other programmable data processing apparatus, such as in a processor or processing system (shown as 300 in Fig. 14), to function in a particular manner, such that the instructions stored in the computer-readable memory produce an article of manufacture including computer-readable instructions for implementing the function specified in the blocks illustrated in Fig. 14. The computer program instructions may also be loaded onto a computer, the processing system 300, or other programmable data processing apparatus to cause a series of operational steps to be performed on the computer, the processing system 300, or other programmable apparatus to produce a computer-implemented process such that the instructions that execute on the computer or other programmable apparatus provide steps for implementing the functions specified in the blocks. Moreover, the various software modules 310, 320, and 330 used to perform the various calculations and perform related method steps described herein also can be stored as computer-executable instructions on a computer-readable medium in order to allow the methods to be loaded into and executed by different processing systems.

Accordingly, blocks of the block diagrams and flowchart illustrations support combinations of means for performing the specified functions, combinations of steps for performing the specified functions, and program instruction means for performing the specified functions. One of skill will understand that each block of the block diagrams and flowchart illustrations, and combinations of blocks in the block diagrams and flowchart illustrations, can be implemented by special purpose hardware-based computer systems that perform the specified functions or steps, or combinations of special purpose hardware and computer instructions.

The present invention is not limited in scope by the embodiments disclosed herein which are intended as illustrations of a few aspects of the invention.

**Claims**

1. A computer program product for determining a cardiovascular parameter in a subject, the computer program product comprising computer program instructions stored on a computer-readable medium which, when loaded into and executed by a processing system (300), cause the processing system (300) to execute the following steps:

   receiving arterial pressure waveform data from a subject;
   analyzing the arterial pressure waveform data from the subject to determine if the subject is experiencing a hyperdynamic hemodynamic condition;
   if the subject is determined to be experiencing the hyperdynamic hemodynamic condition, then applying a second multivariate statistical model to the arterial pressure waveform data to determine the subject's cardiovascular parameter; and
   if the subject is not determined to be experiencing the hyperdynamic hemodynamic condition, then applying a third multivariate statistical model to the arterial pressure waveform data to determine the subject's cardiovascular parameter;
   wherein determining if the subject is experiencing the hyperdynamic hemodynamic condition comprises applying a first multivariate statistical model to the arterial pressure waveform data to determine if the subject is experiencing the hyperdynamic hemodynamic condition, the first multivariate statistical model being prepared from a first set of arterial pressure waveform data from a first group of test subjects that were experiencing the hyperdynamic hemodynamic condition and a second set of arterial pressure waveform data from a second group of test subjects that were not experiencing the hyperdynamic hemodynamic condition, the first multivariate statistical model providing an output value that corresponds to a first value for the arterial pressure waveforms of the first set of arterial pressure waveform data and a second value for the arterial pressure waveforms of the second set of arterial pressure waveform data, wherein if the output value is greater than a threshold value between the first value and second value, then the subject is determined to be experiencing the hyperdynamic hemodynamic condition; and
   wherein determining if the subject is experiencing the hyperdynamic hemodynamic condition comprises applying the first multivariate statistical model using the following steps:

      determining a first approximating function relating the first set of arterial pressure waveform data from the first group of test subjects that were experiencing the hyperdynamic hemodynamic condition and the second set of arterial pressure waveform data from the second group of test subjects that were not experiencing the hyperdynamic hemodynamic condition,
      determining a first set of arterial blood pressure parameters from the arterial blood pressure waveform data, and
      determining if the subject is experiencing the hyperdynamic hemodynamic condition by evaluating the first approximating function with the first set of arterial blood pressure parameters.

2. The computer program product of claim 1,
   wherein the first approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area;
and wherein the first set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area.

3. The computer program product of claim 1, wherein the second multivariate statistical model is prepared from a set of arterial pressure waveform data from a group of test subjects that were experiencing the hyperdynamic hemodynamic condition, the second multivariate statistical model providing a value for the subject's cardiovascular parameter.

4. The computer program product of claim 3, wherein the second multivariate statistical model is based on a set of factors including one or more parameters affected by the hyperdynamic hemodynamic condition.

5. The computer program product of claim 1, wherein the third multivariate statistical model is prepared from a set of arterial pressure waveform data from a group of test subjects that were not experiencing the hyperdynamic hemodynamic condition, the third multivariate statistical model providing a value for the subject's normal cardiovascular parameter.

6. The computer program product of claim 5, wherein the third multivariate statistical model is based on a set of factors including one or more parameters used to calculate the cardiovascular parameter.

7. The computer program product of claim 1, wherein the subject's cardiovascular parameter is determined by applying the second multivariate statistical model using the following steps:

determining a second approximating function relating a set of clinically derived reference measurements representing blood pressure parameters dependent upon the cardiovascular parameter, the second approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area, and a set of clinically determined reference measurements representing blood pressure parameters dependent upon the cardiovascular parameter from subjects experiencing hyperdynamic hemodynamic conditions;
determining a second set of arterial blood pressure parameters from the arterial blood pressure waveform data, the second set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and

estimating the subject's cardiovascular parameter by evaluating the second approximating function with the second set of arterial blood pressure parameters.

8. The computer program product of claim 1, wherein the subject's normal cardiovascular parameter is determined by applying the third multivariate statistical model using the following steps:

determining a third approximating function relating a set of clinically derived reference measurements representing blood pressure parameters dependent upon the cardiovascular parameter, the third approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area, and a set of clinically determined reference measurements representing blood pressure parameters dependent upon the cardiovascular parameter from subjects not experiencing the hyperdynamic hemodynamic conditions;

determining a third set of arterial blood pressure parameters from the arterial blood pressure waveform data, the third set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and

estimating the subject's normal hemodynamic cardiovascular parameter by evaluating the third approximating function with the third set of arterial blood pressure parameters.

9. The computer program product of any of claims 4 or 6, wherein the one or more parameters are selected from the group consisting of (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, and (m) a parameter based on the pressure dependent Windkessel compliance of a set of arterial pressure waveform data.

10. A processing system (300) for calculating a cardiovascular parameter in a subject, comprising one or more processors and arranged to receive arterial pressure waveform data from the subject;
**characterized in that** the processing system (300) is further arranged to implement the following steps:

analyzing the arterial pressure waveform data to determine if the subject is experiencing a hyperdynamic hemodynamic condition;
if the subject is determined to be experiencing the hyperdynamic hemodynamic condition, then applying a second multivariate statistical model to the arterial pressure waveform data to determine the subject's cardiovascular parameter; and
if the subject is not determined to be experiencing the hyperdynamic hemodynamic condition, then applying a third multivariate statistical model to the arterial pressure waveform data to determine the subject's cardiovascular parameter;
wherein determining if the subject is experiencing the hyperdynamic hemodynamic condition comprises applying a first multivariate statistical model to the arterial pressure waveform data to determine if the subject is experiencing the hyperdynamic hemodynamic condition, the first multivariate statistical model being prepared from a first set of arterial pressure waveform data from a first group of test subjects that were experiencing the hyperdynamic hemodynamic condition and a second set of arterial pressure waveform data from a second group of test subjects that were not experiencing the hyperdynamic hemodynamic condition, the first multivariate statistical model providing an output value that corresponds to a first value for the arterial pressure waveforms of the first set of arterial pressure waveform data and a second value for the arterial pressure waveforms of the second set of arterial pressure waveform data, wherein if the output value is greater than a threshold value between the first value and second value, then the subject is determined to be experiencing the hyperdynamic hemodynamic condition; and
wherein determining if the subject is experiencing the hyperdynamic hemodynamic condition comprises applying the first multivariate statistical model using the following steps:

determining a first approximating function relating the first set of arterial pressure waveform data from the first group of test subjects that were experiencing the hyperdynamic hemodynamic condition and the second set of arterial pressure waveform data from the second group of test subjects that were not experiencing the hyperdynamic hemodynamic condition,
determining a first set of arterial blood pressure parameters from the arterial blood pressure waveform data, and
determining if the subject is experiencing the hyperdynamic hemodynamic condition by evaluating the first approximating function with the first set of arterial blood pressure parameters.

11. The processing system (300) of claim 10,
wherein the first approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and wherein the first set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area.

12. The processing system (300) of claim 10, wherein the second multivariate statistical model is prepared from a set of arterial pressure waveform data from a group of test subjects that were experiencing the hyperdynamic hemodynamic condition, the second multivariate statistical model providing a value for the subject's cardiovascular parameter.

13. The processing system (300) of claim 12, wherein the second multivariate statistical model is based on a set of factors including one or more parameters affected by the hyperdynamic hemodynamic condition.

14. The processing system (300) of claim 10, wherein the third multivariate statistical model is prepared from a set of arterial pressure waveform data from a group of test subjects that were not experiencing the hyperdynamic hemodynamic condition, the third multivariate statistical model providing a value for the subject's normal cardiovascular parameter.

15. The processing system (300) of claim 14, wherein the third multivariate statistical model is based on a set of factors including one or more parameters used to calculate the cardiovascular parameter.

16. The processing system (300) of claim 10, wherein the subject's cardiovascular parameter is determined by applying the second multivariate statistical model using the following steps:

determining a second approximating function relating a set of clinically derived reference measurements representing blood pressure parameters dependent upon the cardiovascular parameter, the second approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area, and a set of clinically determined reference measurements representing blood pressure parameters dependent upon the cardiovascular parameter from subjects experiencing hyperdynamic hemodynamic conditions; determining a second set of arterial blood pressure parameters from the arterial blood pressure waveform data, the second set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and estimating the subject's cardiovascular parameter by evaluating the second approximating function with the second set of arterial blood pressure parameters.

17. The processing system (300) of claim 10, wherein the subject's normal cardiovascular parameter is determined by applying the third multivariate statistical model using the following steps:

determining a third approximating function relating a set of clinically derived reference measurements representing blood pressure parameters dependent upon the cardiovascular parameter, the third approximating function being a function of at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area, and a set of clinically determined reference measurements representing blood pressure parameters dependent upon the cardiovascular parameter from subjects not experiencing the hyperdynamic hemodynamic conditions;

determining a third set of arterial blood pressure parameters from the arterial blood pressure waveform data, the third set of arterial blood pressure parameters including at least (a) a parameter based on the standard deviation of the arterial pressure waveform data, (b) a parameter based on the subject's heart rate, (c) a parameter based on the area under the systolic portion of the arterial blood pressure signal, (d) a parameter based on the duration of systole, (e) a parameter based on the ratio of the duration of the systole to the duration of the diastole, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, (m) a parameter based on the pressure dependent Windkessel compliance of a set of arterial pressure waveform data, and (n) a parameter based on the subject's body surface area; and

estimating the subject's normal hemodynamic cardiovascular parameter by evaluating the third approximating function with the third set of arterial blood pressure parameters.

18. The processing system (300) of any of claims 13 or 15, wherein the one or more parameters are selected from the group consisting of (a) a parameter based on the pulse beats standard deviation of a set of arterial pressure waveform data, (b) a parameter based on the R-to-R interval of a set of arterial pressure waveform data, (c) a parameter based on the area under the systolic portion of a set of arterial pressure waveform data, (d) a parameter based on the duration of systole of a set of arterial pressure waveform data, (e) a parameter based on the duration of the diastole of a set of arterial pressure waveform data, (f) a parameter based on the mean arterial pressure of a set of arterial pressure waveform data, (g) a parameter based on the pressure weighted standard deviation of a set of arterial pressure waveform data, (h) a parameter based on the pressure weighted mean of a set of arterial pressure waveform data, (i) a parameter based on the arterial pulse beats skewness values of a set of arterial pressure waveform data, (j) a parameter based on the arterial pulse beats kurtosis values of a set of arterial pressure waveform data, (k) a parameter based on the pressure weighted skewness of a set of arterial pressure waveform data, (l) a parameter based on the pressure weighted kurtosis of a set of arterial pressure waveform data, and (m) a parameter based on the pressure dependent Windkessel compliance of a set of arterial pressure waveform data.

19. A system for calculating a cardiovascular parameter in a subject, comprising:

a processing system (300) according to one of the claims 10 to 18, and
at least one sensor (100, 200) arranged to yield an electric signal corresponding to the arterial blood pressure, wherein the signal of the at least one sensor (100, 200) is passed as input to the processing system (300).

**Patentansprüche**

1. Computerprogramm zum Ermitteln eines kardiovaskulären Parameters einer Versuchsperson, wobei das Computerprogramm Computerprogrammanweisungen umfasst, die auf einem computerlesbaren Medium gespeichert sind, die, wenn sie in ein Verarbeitungssystem (300) geladen und von diesem ausgeführt werden, bewirken, dass das Verarbeitungssystem (300) die folgenden Schritte ausführt:

Empfangen der Wellenformdaten des arteriellen Drucks von einer Versuchsperson;

Analysieren der Wellenformdaten des arteriellen Drucks der Versuchsperson, um zu ermitteln, ob die Versuchsperson sich in einem hyperdynamischen hämodynamischen Zustand befindet;

wenn ermittelt wird, dass sich die Versuchsperson im hyperdynamischen hämodynamischen Zustand befindet, dann Anwenden eines zweiten multivariaten statistischen Modells auf die Wellenformdaten des arteriellen Drucks, um den kardiovaskulären Parameter der Versuchsperson zu ermitteln; und

wenn nicht ermittelt wird, dass sich die Versuchsperson im hyperdynamischen hämodynamischen Zustand befindet, dann Anwenden eines dritten multivariaten statistischen Modells auf die Wellenformdaten des arteriellen Drucks, um den kardiovaskulären Parameter der Versuchsperson zu ermitteln:

wobei das Ermitteln, ob die Versuchsperson sich im hyperdynamischen hämodynamischen Zustand befindet, das Anwenden eines ersten multivariaten statistischen Modells auf die Wellenformdaten des arteriellen Drucks umfasst, um zu ermitteln, ob die Versuchsperson sich im hyperdynamischen hämodynamischen Zustand befindet, wobei das erste multivariate statistische Modell aus einem ersten Satz Wellenformdaten des arteriellen Drucks von einer ersten Gruppe von Versuchspersonen, die sich im hyperdynamischen hämodynamischen Zustand befanden, und einem zweiten Satz von Wellenformdaten des arteriellen Drucks von einer zweiten Gruppe von Versuchspersonen, die sich nicht im hyperdynamischen hämodynamischen Zustand befanden, erstellt wird, wobei das erste multivariate statistische Modell einen Ausgangswert bereitstellt, der einem ersten Wert für die Wellenformen des arteriellen Drucks des ersten Satzes von Wellenformdaten des arteriellen Drucks und einem zweiten Wert für die Wellenformen des arteriellen Drucks des zweiten Satzes von Wellenformdaten des arteriellen Drucks entspricht, wobei, wenn der Ausgangswert höher ist als ein Schwellenwert zwischen dem ersten Wert und dem zweiten Wert, ermittelt wird, dass sich die Versuchsperson im hyperdynamischen hämodynamischen Zustand befindet; und

wobei das Ermitteln, ob die Versuchsperson sich im hyperdynamischen hämodynamischen Zustand befindet, das Anwenden des ersten multivariaten statistischen Modells unter Verwendung der folgenden Schritte umfasst:

Ermitteln einer ersten approximierenden Funktion, die den ersten Satz Wellenformdaten des arteriellen Drucks von der ersten Gruppe von Versuchspersonen, die sich im hyperdynamischen hämodynamischen Zustand befanden, und den zweiten Satz Wellenformdaten des arteriellen Drucks von der zweiten Gruppe von Versuchspersonen, die sich nicht im hyperdynamisch hämodynamischen Zustand befanden, in Beziehung setzt,

Ermitteln eines ersten Satzes von arteriellen Blutdruckparametern aus den Wellenformdaten des arteriellen Blutdrucks und

Ermitteln, ob die Versuchsperson sich im hyperdynamischen hämodynamischen Zustand befindet, durch Analysieren der ersten approximierenden Funktion mit dem ersten Satz arterieller Blutdruckparameter.

2. Computerprogramm nach Anspruch 1,

wobei die erste approximierende Funktion eine Funktion ist von mindestens (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson;

und wobei der erste Satz arterieller Blutdruckparameter mindestens Folgendes beinhaltet (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson.

3. Computerprogramm nach Anspruch 1, wobei das zweite multivariate statistische Modell aus einem Satz von Wellenformdaten des arteriellen Drucks von einer Gruppe Versuchspersonen erstellt wird, die sich im hyperdynamischen hämodynamischen Zustand befanden, wobei das zweite multivariate statistische Modell einen Wert für den kardiovaskulären Parameter der Versuchsperson bereitstellt.

4. Computerprogramm nach Anspruch 3, wobei das zweite multivariate statistische Modell auf einem Satz von Faktoren basiert, der einen oder mehrere Parameter beinhaltet, die vom hyperdynamischen hämodynamischen Zustand beeinflusst werden.

5. Computerprogramm nach Anspruch 1, wobei das dritte multivariate statistische Modell aus einem Satz von Wellenformdaten des arteriellen Drucks von einer Gruppe von Versuchspersonen erstellt wird, die sich nicht im hyperdynamischen hämodynamischen Zustand befanden, wobei das dritte multivariate statistische Modell einen Wert für den normalen kardiovaskulären Parameter der Versuchsperson bereitstellt.

6. Computerprogramm nach Anspruch 5, wobei das dritte multivariate statistische Modell auf einem Satz von Faktoren basiert, der einen oder mehrere Parameter beinhaltet, die verwendet werden, um den kardiovaskulären Parameter zu berechnen.

7. Computerprogramm nach Anspruch 1, wobei der kardiovaskuläre Parameter der Versuchsperson durch Anwenden des zweiten multivariaten statistischen Modells unter Verwendung der folgenden Schritte ermittelt wird:

Ermitteln einer zweiten approximierenden Funktion, die einen Satz klinisch abgeleiteter Referenzmessungen, die Blutdruckparameter darstellen, die vom kardiovaskulären Parameter abhängen, wobei die zweite approximierende Funktion eine Funktion ist von mindestens (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson, und einen Satz klinisch ermittelter Referenzmessungen, die Blutdruckparameter darstellen, die vom kardiovaskulären Parameter von Versuchspersonen abhängen, die sich in hyperdynamischen hämodynamischen Zuständen befinden, in Beziehung zueinander setzt;

Ermitteln eines zweiten Satzes arterieller Blutdruckparameter aus den Wellenformdaten des arteriellen Blutdrucks, wobei der zweite Satz von arteriellen Blutdruckparametern mindestens Folgendes beinhaltet: (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson; und

Schätzen des kardiovaskulären Parameters der Versuchsperson durch Auswerten der zweiten approximierenden Funktion mit dem zweiten Satz von arteriellen Blutdruckparametern.

8. Computerprogramm nach Anspruch 1, wobei der normale kardiovaskuläre Parameter der Versuchsperson durch Anwenden des dritten multivariaten statistischen Modells unter Verwendung der folgenden Schritte ermittelt wird:

Ermitteln einer dritten approximierenden Funktion, die einen Satz klinisch abgeleiteter Referenzmessungen, die Blutdruckparameter darstellen, die vom kardiovaskulären Parameter abhängen, wobei die dritte approximierende Funktion eine Funktion ist von mindestens (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson, und einen Satz klinisch ermittelter Referenzmessungen, die Blutdruckparameter darstellen, die vom kardiovaskulären Parameter von Versuchspersonen abhängen, die sich nicht in hyperdynamischen hämodynamischen Zuständen befinden, in Beziehung zueinander setzt;

Ermitteln eines dritten Satzes von arteriellen Blutdruckparametern aus den Wellenformdaten des arteriellen Blutdrucks, wobei der dritte Satz von arteriellen Blutdruckparametern mindestens Folgendes beinhaltet: (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson; und

Schätzen des normalen hämodynamischen kardiovaskulären Parameters der Versuchsperson durch Auswerten der dritten approximierenden Funktion mit dem dritten Satz von arteriellen Blutdruckparametern.

9. Computerprogramm nach einem der Ansprüche 4 oder 6, wobei der eine oder mehrere Parameter aus der Gruppe ausgewählt ist, die aus Folgendem besteht: (a) einem Parameter basierend auf der Standardabweichung der Pulsschläge eines Satzes von Wellenformdaten des arteriellen Drucks, (b) einem Parameter basierend auf dem RR-Abstand eines Satzes von Wellenformdaten des arteriellen Drucks, (c) einem Parameter basierend auf dem Bereich unter dem systolischen Abschnitt eines Satzes von Wellenformdaten des arteriellen Drucks, (d) einem Parameter basierend auf der Dauer der Systole eines Satzes von Wellenformdaten des arteriellen Drucks, (e) einem Parameter basierend auf der Dauer der Diastole eines Satzes von Wellenformdaten des arteriellen Drucks, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks.

10. Verarbeitungssystem (300) zur Berechnung eines kardiovaskulären Parameters einer Versuchsperson, das einen oder mehrere Prozessoren umfasst und so angeordnet ist, dass es Wellenformdaten des arteriellen Drucks von der Versuchsperson empfängt;

**dadurch gekennzeichnet, dass** das Verarbeitungssystem (300) ferner so angeordnet ist, dass es die folgenden Schritte umsetzt:

Analysieren der Wellenformdaten des arteriellen Drucks, um zu ermitteln, ob die Versuchsperson sich in einem hyperdynamischen hämodynamischen Zustand befindet;

wenn ermittelt wird, dass sich die Versuchsperson im hyperdynamischen hämodynamischen Zustand befindet, dann Anwenden eines zweiten multivariaten statistischen Modells auf die Wellenformdaten des arteriellen Drucks, um den kardiovaskulären Parameter der Versuchsperson zu ermitteln; und

wenn nicht ermittelt wird, dass sich die Versuchsperson im hyperdynamischen hämodynamischen Zustand befindet, dann Anwenden eines dritten multivariaten statistischen Modells auf die Wellenformdaten des arteriellen Drucks, um den kardiovaskulären Parameter der Versuchsperson zu ermitteln:

wobei das Ermitteln, ob die Versuchsperson sich im hyperdynamischen hämodynamischen Zustand befindet, das Anwenden eines ersten multivariaten statistischen Modells auf die Wellenformdaten des arteriellen Drucks umfasst, um zu ermitteln, ob die Versuchsperson sich im hyperdynamischen hämodynamischen Zustand befindet, wobei das erste multivariate statistische Modell aus einem ersten Satz Wellenformdaten des arteriellen Drucks von einer ersten Gruppe von Versuchspersonen, die sich im hyperdynamischen hämodynamischen Zustand befanden, und einem zweiten Satz von Wellenformdaten des arteriellen Drucks von einer zweiten Gruppe von Versuchspersonen, die sich nicht im hyperdynamischen hämodynamischen Zustand befanden, erstellt wird, wobei das erste multivariate statistische Modell einen Ausgangswert bereitstellt, der einem ersten Wert für die Wellenformen des arteriellen Drucks des ersten Satzes von Wellenformdaten des arteriellen Drucks und einem zweiten Wert für die Wellenformen des arteriellen Drucks des zweiten Satzes von Wellenformdaten des arteriellen Drucks entspricht, wobei, wenn der Ausgangswert höher ist als ein Schwellenwert zwischen dem ersten Wert und dem zweiten Wert, ermittelt wird, dass sich die Versuchsperson im hyperdynamischen hämodynamischen Zustand befindet; und

wobei das Ermitteln, ob die Versuchsperson sich im hyperdynamischen hämodynamischen Zustand befindet, das Anwenden des ersten multivariaten statistischen Modells unter Verwendung der folgenden Schritte umfasst:

Ermitteln einer ersten approximierenden Funktion, die den ersten Satz Wellenformdaten des arteriellen Drucks von der ersten Gruppe von Versuchspersonen, die sich im hyperdynamischen hämodynamischen Zustand befanden, und den zweiten Satz Wellenformdaten des arteriellen Drucks von der zweiten Gruppe von Versuchspersonen, die sich nicht im hyperdynamisch hämodynamischen Zustand befanden, in Beziehung setzt,

Ermitteln eines ersten Satzes von arteriellen Blutdruckparametern aus den Wellenformdaten des arteriellen Blutdrucks und

Ermitteln, ob die Versuchsperson sich im hyperdynamischen hämodynamischen Zustand befindet, durch Analysieren der ersten approximierenden Funktion mit dem ersten Satz arterieller Blutdruckparameter.

11. Verarbeitungssystem (300) nach Anspruch 10,

wobei die erste approximierende Funktion eine Funktion ist von mindestens (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson;

und wobei der erste Satz arterieller Blutdruckparameter mindestens Folgendes beinhaltet (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson.

12. Verarbeitungssystem (300) nach Anspruch 10, wobei das zweite multivariate statistische Modell aus einem Satz von Wellenformdaten des arteriellen Drucks von einer Gruppe von Versuchspersonen erstellt wird, die sich im hyperdynamischen hämodynamischen Zustand befanden, wobei das zweite multivariate statistische Modell einen Wert für den kardiovaskulären Parameter der Versuchsperson bereitstellt.

13. Verarbeitungssystem (300) nach Anspruch 12, wobei das zweite multivariate statistische Modell auf einem Satz von Faktoren basiert, der einen oder mehrere Parameter beinhaltet, die vom hyperdynamischen hämodynamischen Zustand beeinflusst werden.

**14.** Verarbeitungssystem (300) nach Anspruch 10, wobei das dritte multivariate statistische Modell aus einem Satz von Wellenformdaten des arteriellen Drucks von einer Gruppe von Versuchspersonen erstellt wird, die sich nicht im hyperdynamischen hämodynamischen Zustand befanden, wobei das dritte multivariate statistische Modell einen Wert für den normalen kardiovaskulären Parameter der Versuchsperson bereitstellt.

**15.** Verarbeitungssystem (300) nach Anspruch 14, wobei das dritte multivariate statistische Modell auf einem Satz von Faktoren basiert, der einen oder mehrere Parameter beinhaltet, die zum Berechnen des kardiovaskulären Parameters verwendet werden.

**16.** Verarbeitungssystem (300) nach Anspruch 10, wobei der kardiovaskuläre Parameter der Versuchsperson ermittelt wird, indem das zweite multivariate statistische Modell unter Verwendung der folgenden Schritte angewandt wird:

Ermitteln einer zweiten approximierenden Funktion, die einen Satz klinisch abgeleiteter Referenzmessungen, die Blutdruckparameter darstellen, die vom kardiovaskulären Parameter abhängen, wobei die zweite approximierende Funktion eine Funktion ist von mindestens (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson, und einen Satz klinisch ermittelter Referenzmessungen, die Blutdruckparameter darstellen, die vom kardiovaskulären Parameter von Versuchspersonen abhängen, die sich in hyperdynamischen hämodynamischen Zuständen befinden, in Beziehung zueinander setzt;
Ermitteln eines zweiten Satzes arterieller Blutdruckparameter aus den Wellenformdaten des arteriellen Blutdrucks, wobei der zweite Satz von arteriellen Blutdruckparametern mindestens Folgendes beinhaltet: (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson; und
Schätzen des kardiovaskulären Parameters der Versuchsperson durch Auswerten der zweiten approximierenden Funktion mit dem zweiten Satz von arteriellen Blutdruckparametern.

**17.** Verarbeitungssystem (300) nach Anspruch 10, wobei der normale kardiovaskuläre Parameter der Versuchsperson ermittelt wird, indem das dritte multivariate statistische Modell unter Verwendung der folgenden Schritte angewandt wird:

Ermitteln einer dritten approximierenden Funktion, die einen Satz klinisch abgeleiteter Referenzmessungen, die Blutdruckparameter darstellen, die vom kardiovaskulären Parameter abhängen, wobei die dritte approximierende Funktion eine Funktion ist von mindestens (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson, und einen Satz klinisch ermittelter Referenzmessungen, die Blutdruckparameter darstellen, die vom kardiovaskulären Parameter von Versuchspersonen abhängen, die sich nicht in hyperdynamischen hämodynamischen Zuständen befinden, in Beziehung zueinander setzt;

Ermitteln eines dritten Satzes von arteriellen Blutdruckparametern aus den Wellenformdaten des arteriellen Blutdrucks, wobei der dritte Satz von arteriellen Blutdruckparametern mindestens Folgendes beinhaltet: (a) einem Parameter basierend auf der Standardabweichung der Wellenformdaten des arteriellen Drucks, (b) einem Parameter basierend auf der Herzfrequenz der Versuchsperson, (c) einem Parameter basierend auf dem Bereich unter dem systolischen Abschnitt des arteriellen Blutdrucksignals, (d) einem Parameter basierend auf der Dauer der Systole, (e) einem Parameter basierend auf dem Verhältnis der Dauer der Systole zur Dauer der Diastole, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks, und (n) einem Parameter basierend auf der Körperoberfläche der Versuchsperson; und

Schätzen des normalen hämodynamischen kardiovaskulären Parameters der Versuchsperson durch Auswerten der dritten approximierenden Funktion mit dem dritten Satz von arteriellen Blutdruckparametern.

**18.** Verarbeitungssystem (300) nach einem der Ansprüche 13 oder 15, wobei der eine oder mehrere Parameter aus der Gruppe ausgewählt ist, die aus Folgendem besteht: (a) einem Parameter basierend auf der Standardabweichung der Pulsschläge eines Satzes von Wellenformdaten des arteriellen Drucks, (b) einem Parameter basierend auf dem RR-Abstand eines Satzes von Wellenformdaten des arteriellen Drucks, (c) einem Parameter basierend auf dem Bereich unter dem systolischen Abschnitt eines Satzes von Wellenformdaten des arteriellen Drucks, (d) einem Parameter basierend auf der Dauer der Systole eines Satzes von Wellenformdaten des arteriellen Drucks, (e) einem Parameter basierend auf der Dauer der Diastole eines Satzes von Wellenformdaten des arteriellen Drucks, (f) einem Parameter basierend auf dem mittleren arteriellen Druck eines Satzes von Wellenformdaten des arteriellen Drucks, (g) einem Parameter basierend auf der druckgewichteten Standardabweichung eines Satzes von Wellenformdaten des arteriellen Drucks, (h) einem Parameter basierend auf dem druckgewichteten Mittel eines Satzes von Wellenformdaten des arteriellen Drucks, (i) einem Parameter basierend auf den Schiefewerten der arteriellen Pulsschläge eines Satzes von Wellenformdaten des arteriellen Drucks, (j) einem Parameter basierend auf den Wölbungswerten der arteriellen Pulsschläge eines Satzes von Wellenformdaten des arteriellen Drucks, (k) einem Parameter basierend auf der druckgewichteten Schiefe eines Satzes von Wellenformdaten des arteriellen Drucks, (l) einem Parameter basierend auf der druckgewichteten Wölbung eines Satzes von Wellenformdaten des arteriellen Drucks, (m) einem Parameter basierend auf der druckabhängigen Windkessel-Compliance eines Satzes von Wellenformdaten des arteriellen Drucks.

**19.** System zur Berechnung eines kardiovaskulären Parameters einer Versuchsperson, umfassend:

ein Verarbeitungssystem (300) nach einem der Ansprüche 10 bis 18, und

mindestens einen Sensor (100, 200), der so angeordnet ist, dass er ein elektrisches Signal liefert, das dem arteriellen Blutdruck entspricht,

wobei das Signal von dem mindestens einen Sensor (100, 200) als Eingangssignal an das Verarbeitungssystem (300) weitergegeben wird.

**Revendications**

1.  Un produit-programme informatique pour déterminer un paramètre cardiovasculaire chez un sujet, le produit-programme informatique comprenant les instructions du programme informatique enregistrées sur un support lisible par un ordinateur qui, lorsqu'il est inséré dans et exécuté par un système de traitement (300), entraîne l'exécution des étapes suivantes par le système de traitement (300) :

    réception de données de forme d'onde de pression artérielle d'un sujet ;

    analyse des données de forme d'onde de pression artérielle du sujet pour déterminer si le sujet présente un état hémodynamique hyperdynamique ;

    si le sujet est déterminé comme présentant l'état hémodynamique hyperdynamique, application d'un deuxième modèle statistique multivarié aux données de forme d'onde de pression artérielle pour déterminer le paramètre cardiovasculaire du sujet ; et

    si le sujet n'est pas déterminé comme présentant l'état hémodynamique hyperdynamique, application d'un troisième modèle statistique multivarié aux données de forme d'onde de pression artérielle pour déterminer le paramètre cardiovasculaire du sujet ;

    la détermination de si oui ou non le sujet présente l'état hémodynamique hyperdynamique comprenant l'application d'un premier modèle statistique multivarié aux données de forme d'onde de pression artérielle pour déterminer si le sujet présente l'état hémodynamique hyperdynamique, le premier modèle statistique multivarié étant préparé à partir d'un premier ensemble de données de forme d'onde de pression artérielle d'un premier groupe de sujets testés ayant présenté l'état hémodynamique hyperdynamique et d'un deuxième ensemble de données de forme d'onde de pression artérielle d'un deuxième groupe de sujets testés n'ayant pas présenté l'état hémodynamique hyperdynamique, le premier modèle statistique multivarié fournissant une valeur de sortie correspondant à une première valeur pour les formes d'onde de pression artérielle du premier ensemble de données de forme d'onde de pression artérielle et à une deuxième valeur pour les formes d'onde de pression artérielle du deuxième ensemble de données de forme d'onde de pression artérielle, le sujet étant déterminé comme présentant l'état hémodynamique hyperactive si la valeur de sortie est supérieure à une valeur limite située entre la première et la deuxième valeurs ; et

    la détermination de si oui ou non le sujet présente l'état hémodynamique hyperdynamique comprenant l'application du premier modèle statistique multivarié selon les étapes suivantes :

       détermination d'une première fonction d'approximation reliant le premier ensemble de données de forme d'onde de pression artérielle du premier groupe de sujets testés ayant présenté l'état hémodynamique hyperdynamique et le deuxième ensemble de données de forme d'onde de pression artérielle du deuxième groupe de sujets testés n'ayant pas présenté l'état hémodynamique hyperdynamique,

       détermination d'un premier ensemble de paramètres de pression artérielle à partir des données de forme d'onde de pression artérielle, et

       détermination de si oui ou non le sujet présente l'état hémodynamique hyperdynamique en évaluant la première fonction d'approximation avec le premier ensemble de paramètres de pression artérielle.

2.  Produit-programme informatique selon la revendication 1,

    la première fonction d'approximation étant une fonction d'au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet ;

et le premier ensemble de paramètres de pression artérielle incluant au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet.

3. Produit-programme informatique selon la revendication 1, le deuxième modèle statistique multivarié étant préparé à partir d'un ensemble de données de forme d'onde de pression artérielle d'un groupe de sujets testés ayant présenté l'état hémodynamique hyperdynamique, le deuxième modèle statistique multivarié fournissant une valeur pour le paramètre cardiovasculaire du sujet.

4. Produit-programme informatique selon la revendication 3, le deuxième modèle statistique multivarié étant basé sur un ensemble de facteurs incluant un ou plusieurs paramètres affectés par l'état hémodynamique hyperdynamique.

5. Produit-programme informatique selon la revendication 1, le troisième modèle statistique multivarié étant préparé à partir d'un ensemble de données de forme d'onde de pression artérielle d'un groupe de sujets testés n'ayant pas présenté l'état hémodynamique hyperdynamique, le troisième modèle statistique multivarié fournissant une valeur pour le paramètre cardiovasculaire normal du sujet.

6. Produit-programme informatique selon la revendication 5, le troisième modèle statistique multivarié étant basé sur un ensemble de facteurs incluant un ou plusieurs paramètres utilisés pour calculer le paramètre cardiovasculaire.

7. Produit-programme informatique selon la revendication 1, le paramètre cardiovasculaire du sujet étant déterminé en appliquant le deuxième modèle statistique multivarié selon les étapes suivantes :

détermination d'une deuxième fonction d'approximation reliant un ensemble de mesures de référence obtenues cliniquement représentant des paramètres de pression artérielle dépendant du paramètre cardiovasculaire, la deuxième fonction d'approximation étant une fonction d'au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet, et un ensemble de mesures de référence obtenues cliniquement représentant des paramètres de pression artérielle dépendant du paramètre cardiovasculaire des sujets présentant des états hémodynamiques hyperdynamiques ;

détermination d'un deuxième ensemble de paramètres de pression artérielle à partir des données de forme d'onde de pression artérielle, le deuxième ensemble de paramètres de pression artérielle incluant au moins (a) paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet ; et

estimation du paramètre cardiovasculaire du sujet en évaluant la deuxième fonction d'approximation avec le deuxième ensemble de paramètres de pression artérielle.

8. Produit-programme informatique selon la revendication 1, le paramètre cardiovasculaire normal du sujet étant déterminé en appliquant le troisième modèle statistique multivarié selon les étapes suivantes :

détermination d'une troisième fonction d'approximation reliant un ensemble de mesures de référence obtenues cliniquement représentant des paramètres de pression artérielle dépendant du paramètre cardiovasculaire, la troisième fonction d'approximation étant une fonction d'au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet, et un ensemble de mesures de référence déterminées cliniquement représentant des paramètres de pression artérielle dépendant du paramètre cardiovasculaire des sujets n'ayant pas présenté les états hémodynamiques hyperdynamiques ;

détermination d'un troisième ensemble de paramètres de pression artérielle à partir des données de forme d'onde de pression artérielle, le troisième ensemble de paramètres de pression artérielle incluant au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet ; et estimation du paramètre cardiovasculaire hémodynamique normal du sujet en évaluant la troisième fonction d'approximation avec le troisième ensemble de paramètres de pression artérielle.

9. Produit-programme informatique selon l'une des revendications 4 ou 6, l'un ou les plusieurs paramètres étant sélectionnés à partir du groupe comprenant (a) un paramètre basé sur l'écart-type du battement d'un ensemble de données de forme d'onde de pression artérielle, (b) un paramètre basé sur l'intervalle R à R d'un ensemble de données de forme d'onde de pression artérielle, (c) un paramètre basé sur la zone sous la partie systolique d'un ensemble de données de forme d'onde de pression artérielle, (d) un paramètre basé sur la durée de systole d'un ensemble de données de forme d'onde de pression artérielle, (e) un paramètre basé sur la durée de diastole d'un ensemble de données de forme d'onde de pression artérielle, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, et (m) un paramètre basé sur la conformité de l'effet Windkessel dépendant de la pression d'un ensemble de données de forme d'onde de pression artérielle.

10. Un système de traitement (300) pour calculer un paramètre cardiovasculaire chez un sujet, comprenant un ou plusieurs processeurs et conçu pour recevoir des données de forme d'onde de pression artérielle du sujet ; **caractérisé en ce que** le système de traitement (300) est également conçu pour exécuter les étapes suivantes :

analyse des données de forme d'onde de pression artérielle pour déterminer si le sujet présente un état hémodynamique hyperdynamique ;

si le sujet est déterminé comme présentant l'état hémodynamique hyperdynamique, application d'un deuxième modèle statistique multivarié aux données de forme d'onde de pression artérielle pour déterminer le paramètre cardiovasculaire du sujet ; et

si le sujet n'est pas déterminé comme présentant l'état hémodynamique hyperdynamique, application d'un troisième modèle statistique multivarié aux données de forme d'onde de pression artérielle pour déterminer le paramètre cardiovasculaire du sujet ;

la détermination de si oui ou non le sujet présente l'état hémodynamique hyperdynamique comprenant l'application d'un premier modèle statistique multivarié aux données de forme d'onde de pression artérielle pour déterminer si le sujet présente l'état hémodynamique hyperdynamique, le premier modèle statistique multivarié étant préparé à partir d'un premier ensemble de données de forme d'onde de pression artérielle d'un premier groupe de sujets testés ayant présenté l'état hémodynamique hyperdynamique et d'un deuxième ensemble de données de forme d'onde de pression artérielle d'un deuxième groupe de sujets testés n'ayant pas présenté l'état hémodynamique hyperdynamique, le premier modèle statistique multivarié fournissant une valeur de sortie correspondant à une première valeur des formes d'onde de pression artérielle du premier ensemble de données de forme d'onde de pression artérielle et à une deuxième valeur pour les formes d'onde de pression artérielle du deuxième ensemble de données de forme d'onde de pression artérielle, le sujet étant déterminé comme présentant l'état hémodynamique hyperdynamique si la valeur de sortie est supérieure à une valeur limite située entre la première et la deuxième valeurs ; et

la détermination de si oui ou non le sujet présente l'état hémodynamique hyperdynamique comprenant l'application du premier modèle statistique multivarié selon les étapes suivantes :

détermination d'une première fonction d'approximation reliant le premier ensemble de données de forme d'onde de pression artérielle du premier groupe de sujets testés ayant présenté l'état hémodynamique hyperdynamique et le deuxième ensemble de données de forme d'onde de pression artérielle du deuxième groupe de sujets testés n'ayant pas présenté l'état hémodynamique hyperdynamique,

détermination d'un premier ensemble de paramètres de pression artérielle à partir des données de forme d'onde de pression artérielle, et

détermination de si oui ou non le sujet présente l'état hémodynamique hyperdynamique en évaluant la première fonction d'approximation avec le premier ensemble de paramètres de pression artérielle.

**11.** Système de traitement (300) selon la revendication 10,

la première fonction d'approximation étant une fonction d'au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet ;

et le premier ensemble de paramètres de pression artérielle incluant au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet.

**12.** Système de traitement (300) selon la revendication 10, le deuxième modèle statistique multivarié étant préparé à partir d'un ensemble de données de forme d'onde de pression artérielle d'un groupe de sujets testés ayant présenté l'état hémodynamique hyperdynamique, le deuxième modèle statistique multivarié fournissant une valeur pour le paramètre cardiovasculaire du sujet.

**13.** Système de traitement (300) selon la revendication 12, le deuxième modèle statistique multivarié étant basé sur un ensemble de facteurs incluant un ou plusieurs paramètres affectés par l'état hémodynamique hyperdynamique.

**14.** Système de traitement (300) selon la revendication 10, le troisième modèle statistique multivarié étant préparé à partir d'un ensemble de données de forme d'onde de pression artérielle d'un groupe de sujets testés n'ayant pas présenté l'état hémodynamique hyperdynamique, le troisième modèle statistique multivarié fournissant une valeur pour le paramètre cardiovasculaire normal du sujet.

**15.** Système de traitement (300) selon la revendication 14, le troisième modèle statistique multivarié étant basé sur un ensemble de facteurs incluant un ou plusieurs paramètres utilisés pour calculer le paramètre cardiovasculaire.

**16.** Système de traitement (300) selon la revendication 10, le paramètre cardiovasculaire du sujet étant déterminé en appliquant le deuxième modèle statistique multivarié selon les étapes suivantes :

détermination d'une deuxième fonction d'approximation reliant un ensemble de mesures de référence obtenues cliniquement représentant des paramètres de pression artérielle dépendant du paramètre cardiovasculaire, la deuxième fonction d'approximation étant une fonction d'au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet, et un ensemble de mesures de référence déterminées cliniquement représentant des paramètres de pression artérielle dépendant du paramètre cardiovasculaire des sujets présentant des états hémodynamiques hyperdynamiques ;

détermination d'un deuxième ensemble de paramètres de pression artérielle à partir des données de forme d'onde de pression artérielle, le deuxième ensemble de paramètres de pression artérielle incluant au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet ; et estimation du paramètre cardiovasculaire du sujet en évaluant la deuxième fonction d'approximation avec le deuxième ensemble de paramètres de pression artérielle.

17. Système de traitement (300) selon la revendication 10, le paramètre cardiovasculaire normal du sujet étant déterminé en appliquant le troisième modèle statistique multivarié selon les étapes suivantes :

détermination d'une troisième fonction d'approximation reliant un ensemble de mesures de référence obtenues cliniquement représentant des paramètres de pression artérielle dépendant du paramètre cardiovasculaire, la troisième fonction d'approximation étant une fonction d'au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet, et un ensemble de mesures de référence déterminées cliniquement représentant des paramètres de pression artérielle dépendant du paramètre cardiovasculaire des sujets ne présentant pas les états hémodynamiques hyperdynamiques ;

détermination d'un troisième ensemble de paramètres de pression artérielle à partir des données de forme d'onde de pression artérielle, le troisième ensemble de paramètres de pression artérielle incluant au moins (a) un paramètre basé sur l'écart-type des données de forme d'onde de pression artérielle, (b) un paramètre basé sur le rythme cardiaque du sujet, (c) un paramètre basé sur la zone sous la partie systolique du signal de pression artérielle, (d) un paramètre basé sur la durée de systole, (e) un paramètre basé sur le rapport entre la durée de systole et la durée de diastole, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, (m) un paramètre basé sur la conformité de l'effet Windkessel dépendant de la pression d'un ensemble de données de forme d'onde de pression artérielle, et (n) un paramètre basé sur la surface corporelle du sujet ; et estimation du paramètre cardiovasculaire hémodynamique normal du sujet en évaluant la troisième fonction d'approximation avec le troisième ensemble de paramètres de pression artérielle.

18. Système de traitement (300) selon l'une des revendications 13 ou 15, l'un ou les plusieurs paramètres étant sélectionnés à partir du groupe comprenant (a) un paramètre basé sur l'écart-type du battement d'un ensemble de données de forme d'onde de pression artérielle, (b) un paramètre basé sur l'intervalle R à R d'un ensemble de données de forme d'onde de pression artérielle, (c) un paramètre basé sur la zone sous la partie systolique d'un ensemble de données de forme d'onde de pression artérielle, (d) un paramètre basé sur la durée de systole d'un ensemble de données de forme d'onde de pression artérielle, (e) un paramètre basé sur la durée de diastole d'un ensemble de données de forme d'onde de pression artérielle, (f) un paramètre basé sur la pression artérielle moyenne d'un ensemble de données de forme d'onde de pression artérielle, (g) un paramètre basé sur l'écart-type pondéré de la pression d'un ensemble de données de forme d'onde de pression artérielle, (h) un paramètre basé sur la moyenne pondérée de la pression d'un ensemble de données de forme d'onde de pression artérielle, (i) un paramètre basé sur les coefficients d'asymétrie du battement artériel d'un ensemble de données de forme d'onde de pression artérielle, (j) un paramètre basé sur les coefficients d'aplatissement du battement artériel d'un ensemble de données de forme d'onde de pression artérielle, (k) un paramètre basé sur l'asymétrie pondérée de la pression d'un ensemble de données de forme d'onde de pression artérielle, (l) un paramètre basé sur l'aplatissement pondéré de la pression d'un ensemble de données de forme d'onde de pression artérielle, et (m) un paramètre basé sur la conformité de l'effet Windkessel dépendant de la pression d'un ensemble de données de forme d'onde de pression artérielle.

19. Un système pour calculer un paramètre cardiovasculaire chez un sujet comprenant :

un système de traitement (300) selon l'une des revendications 10 à 18, et
au moins un capteur (100, 200) conçu pour générer un signal électrique correspondant à la pression artérielle, le signal de l'au moins un capteur (100, 200) étant transmis comme entrée au système de traitement (300).

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005124904 A1
- US 2008015451 A1
- US 89088704 A
- US 890887 A

**Non-patent literature cited in the description**

- **BENJAMIN PRATT et al.** Calculating Arterial Pressure-Based Cardiac Output Using a Novel Measurement and Analysis Method. *Biomedical Instrumentation & Technology,* September 2007, vol. 41 (5), 403-411
- Continuous and intermittent cardiac output measurement in hyperdynamic conditions: pulmonary artery catheter vs. lithium dilution technique. **MARIA GABRIELLA COSTA et al.** Intensive Care Medicine. Springer, vol. 34, 257-263
- **LANGEWOUTERS et al.** The Static Elastic Properties of 45 Human Thoracic and 20 Abnormal Aortas in vitro and the Parameters of a New Model. *J. Biomechanics,* 1984, vol. 17 (6), 425-435